(19) Europäisches Patentamt
European Patent Office
Office européen des brevets

(11) EP 0 575 490 B1

(12) EUROPEAN PATENT SPECIFICATION

(45) Date of publication and mention of the grant of the patent:
**04.08.1999 Bulletin 1999/31**

(21) Application number: **92908108.1**

(22) Date of filing: **11.03.1992**

(51) Int Cl.$^6$: **C07K 7/23**

(86) International application number:
**PCT/US92/01921**

(87) International publication number:
**WO 92/17025 (01.10.1992 Gazette 1992/25)**

(54) **GnRH ANALOGS**

GnRH Analoge

ANALOGUES DE GnRH

(84) Designated Contracting States:
**AT BE CH DE DK ES FR GB GR IT LI LU NL SE**

(30) Priority: **14.03.1991 US 669695**

(43) Date of publication of application:
**29.12.1993 Bulletin 1993/52**

(73) Proprietor: **THE SALK INSTITUTE FOR BIOLOGICAL STUDIES**
**La Jolla California 92037 (US)**

(72) Inventors:
- **HOEGER, Carl, A.**
  **San Marcos, CA 92069 (US)**
- **RIVIER, Jean, Edouard, Frederic**
  **La Jolla, CA 92037 (US)**
- **THEOBALD, Paula, Guess**
  **Oceanside, CA 92056 (US)**
- **PORTER, John, S.**
  **Leucadia, CA 92024 (US)**
- **RIVIER, Catherine, Laure**
  **La Jolla, CA 92037 (US)**
- **VALE, Wylie, Walker, Jr.**
  **La Jolla, CA 92037 (US)**

(74) Representative:
**Lawrence, Malcolm Graham et al**
**Hepworth, Lawrence, Bryer & Bizley**
**Merlin House**
**Falconry Court**
**Baker's Lane**
**Epping Essex CM16 5DQ (GB)**

(56) References cited:
**EP-A- 0 192 492**
**WO-A-91/06543**
**US-A- 4 234 571**
**US-A- 4 667 014**
**US-A- 4 801 577**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 575 490 B1

## Description

This invention relates generally to peptides having unnatural amino acids and to the preparation of new unnatural amino acids, which may be derived from amino acids having an amino-substituted aromatic side chain, such as para-amino Phe. More particularly, it relates to GnRH analogs having such unnatural amino acids which can be prepared either in such fully assembled peptides or for incorporation into such peptides as a part of the usual chain elongation synthesis process.

In one more particular aspect, the present invention relates to peptides which inhibit gonadal function and the release of the steroidal hormones, progesterone and testosterone, as well as to methods of preventing ovulation.

## BACKGROUND OF THE INVENTION

Follicle stimulating hormone (FSH) and luteinizing hormone (LH), sometimes referred to as gonadotropins or gonadotropic hormones, are released by the pituitary gland. These hormones, in combination, regulate the functioning of the gonads to produce testosterone in the testes and progesterone and estrogen in the ovaries, and they also regulate the production and maturation of gametes.

The release of a hormone by the anterior lobe of the pituitary gland usually requires a prior release of another class of hormones produced by the hypothalamus. The hypothalamic hormone GnRH triggers the release of the gonadotropic hormones, particularly LH; this hormone is also referred to as LH-RH and as LRF. Mammalian GnRH is a well known decapeptide as described in U.S. Patent No. 4,072,668.

Peptides are compounds which contain two or more amino acids in which the carboxyl group of one acid is linked to the amino group of the other acid. In conventional representation of peptides, the amino terminus appears to the left and the carboxyl terminus to the right. The positions of the amino acid residues are identified by numbering the amino acid residues from left to right. In GnRH, the hydroxyl portion of the carboxyl group of glycine at the C-terminus has been replaced with an amino group($NH_2$) i.e. the C-terminus is amidated. The abbreviations for the individual amino acid residues above are conventional and are based on the trivial name of the amino acid, e.g. pGlu is pyroglutamic acid, Glu is glutamic acid, His is histidine, Trp is tryptophan, Ser is serine, Tyr is tyrosine, Gly is glycine, Leu is leucine, Nle is norleucine, Orn is ornithine, Arg is arginine, Har is homoarginine, Pro is proline, Sar is sarcosine, Phe is phenylalanine, Ala is alanine, Val is valine, Nva is norvaline, Ile is isoleucine, Thr is threonine, Lys is lysine, Asp is aspartic acid, Asn is asparagine, Gln is glutamine, and Met is methionine. Except for glycine, amino acids of the peptides of the invention are of the L-configuration unless noted otherwise.

There are reasons for desiring to prevent ovulation in female mammalians, and the administration of GnRH analogs that are antagonistic to the normal function of GnRH have been used to suppress or delay ovulation. For this reason, analogs of GnRH which are antagonistic to GnRH are being investigated for their potential use as a contraceptive or for regulating conception periods. GnRH antagonists may also be used for the treatment of precocious puberty and endometriosis. Such antagonists have also been found useful to regulate the secretion of gonadotropins in male mammals and can be employed to arrest spermatogenesis, e.g. as male contraceptives for treatment of male sex offenders, and for treatment of prostatic hypertrophy. More specifically, GnRH antagonists can be used to treat steroid-dependent tumors, such as prostatic and mammary tumors, and for the control of the timing of ovulation for in vitro fertilization. In the female, they can also be used to treat hirsutism.

In one aspect, it is desired to provide improved peptides which are strongly antagonistic to endogenous GnRH and prevent secretion of LH and FSH and the release of steroids by the gonads of mammals and which have good solubility at physiologically acceptable pH, such as pHs of about 5 to about 6.5. Of particular interest are compounds which are more effective in vivo when administered orally.

## SUMMARY OF THE INVENTION

The present invention provides unnatural amino acids that can be prepared de novo or by modifying a previously prepared peptide, or a protected peptide-resin, containing a desired overall sequence which includes one or more amino acid residues having an amino group which is to be modified. Preferred amino acids of the invention have a side chain which contains either a modified guanidino group or a guanidino equivalent or a derivative that is obtained by further elucidation of a modified guanidino group, as set forth hereinafter.

In another particular aspect, the invention provides peptides which inhibit the release of gonadotropins in mammalians, including humans, and it also provides methods for inhibiting the release of steroids by the gonads of male and female mammalians. As mentioned above, these GnRH antagonists may be used to inhibit the production of gonadotropins and sex hormones under various circumstances, including precocious puberty, hormone dependent neoplasia, dysmenorrhea, endometriosis and steroid-dependent tumors.

The invention provides unnatural amino acids having the following formula U*:

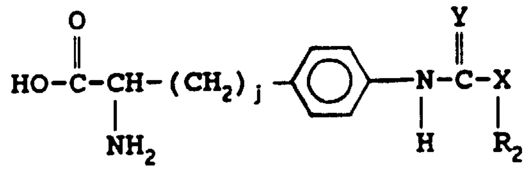

where j is 1,2 or 3; Y = N-CN, N-CONHR$_9$, O, S or CH-NO$_2$, where R$_9$ is H, Ac, alkyl (preferably C$_1$ to C$_4$), naphthyl, pyridyl, pyrimidyl, pyrazinyl, indolyl, quinolinyl or imidazolyl, which alkyl and cyclic groups are unsubstituted or substituted (preferably by chloro, fluoro, bromo, amino, nitro, alkyl (C$_1$ to C$_4$) and alkoxy (C$_1$ to C$_4$)); X = NH, O, N$_3$, M$_1$-(CH$_q$)$_p$-M$_2$ or M$_1$-(CH$_2$)$_{p'}$-M$_2$(CH$_2$)$_{p''}$-M$_3$, where M$_1$ is NR$_{10}$, N, O or CHR$_3$ wherein R$_3$ is methyl, ethyl, propyl, phenyl, pyridinyl, pyrimidinyl or purinyl; q=1 or 2; p, p' and p" are integers between O and 6; R$_{10}$ is H, methyl, ethyl, propyl, phenyl or substituted phenyl (preferably by Cl, F, NO$_2$, or NH$_2$) ; and M$_2$ and M$_3$ = M$_1$, COOH, CONH$_2$, COOR$_3$ or CN (preferably X is NH or O); R$_1$ = H, alkyl (preferably C$_1$ to C$_6$ and most preferably C$_1$ to C$_4$), modified alkyl (preferably C$_1$ to C$_5$, the terminal carbon of which is either substituted with NH$_2$, OH, Cl, Br or F or is replaced with CF$_3$ or CF$_2$CF$_3$), alkenyl (preferably C$_2$ to C$_4$), such as CH$_2$CH=CHR$_3$, alkynyl (preferably C$_2$ to C$_4$), such as CH$_2$C≡CR$_3$, aryl such as benzyl, tolyl, p-aminobenzyl (anilinyl) and pCl-benzyl or a direct bond to X; R$_2$ - R$_1$, OH, NH$_2$, NHR$_1$, heterocycle (preferably as illustrated hereinafter) or desR$_2$, with R$_2$ being desR$_2$ when X = N$_3$. Optionally R$_2$ and X can be interconnected, or R$_1$ and R$_2$ can be connected to each other via a branched or unbranched methylene bridge of type -(CH$_2$)$_m$- or -(CH$_2$)$_m$-M-(CH$_2$)$_{m'}$-. In such an R$_1$-R$_2$ moiety, m and m' are integers from 1 to 6 and preferably from 1 to 3; and M = NH, O, S or CHR$_4$, wherein R$_4$ is lower alkyl or aryl and is preferably methyl, ethyl, propyl, phenyl or pCl-phenyl, with M preferably being O or S. Most preferably, when R$_1$ and R$_2$ are interconnected, they form a 5, 6, or 7-member heterocyclic ring with the "N-C-X" portion of the formula U*. If desired to form a cyclic peptide, XR$_2$ can contain a part of another diamino acid within the same peptide.

Modification of the specified primary amino function of a given amino acid or peptide is carried out by treatment of either the appropriately protected peptide or the amino acid with an appropriate reagent(s). Peptides or amino acids where Y is N-CN (herein referred to as cyanoguanidines) are prepared by reaction of an amino group with diphenyl cyanocarbonimidate (I):

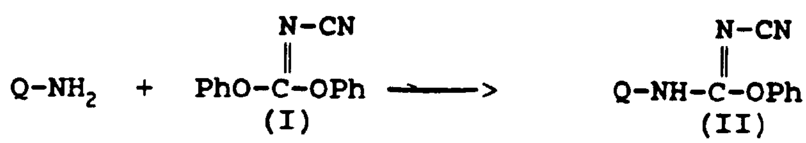

(I) (II)

wherein "Q" is used to broadly represent either the major portion of a peptide or an amino acid having a primary amino group (such as the amino acid which was described above) as a part of formula U*.

The peptide or amino acid having the N-substituted-N'-cyano-O-phenylisourea moiety (II) can then be either isolated or further functionalized by reaction with a second nucleophile HXR$_2$ to produce cyanoguanidine-containing peptides or amino acids having the formula (III):

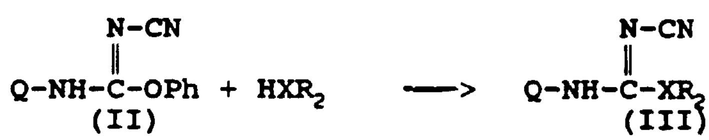

(II) (III)

For example,

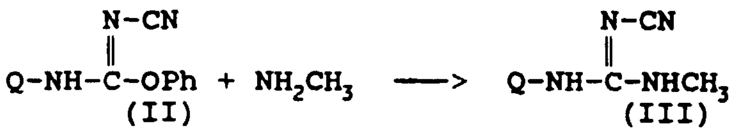

(II) (III) (aminomethyl cyanoguanidino moiety)

For example, where $HXR_2 = H_2N\text{-}CH_2$-pyridine, the result is:

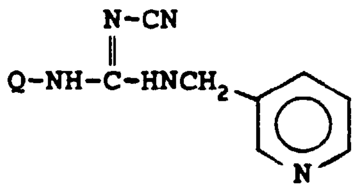

(3 aminomethyl pyridyl cyanoguanidino moiety)

This group may also be referred to (IUPAC nomenclature) as N-g-cyano-N-g'-3-methylpyridylguanidino.

Such compounds can be hydrolyzed under acidic conditions to produce compounds which are also biopotent--for example:

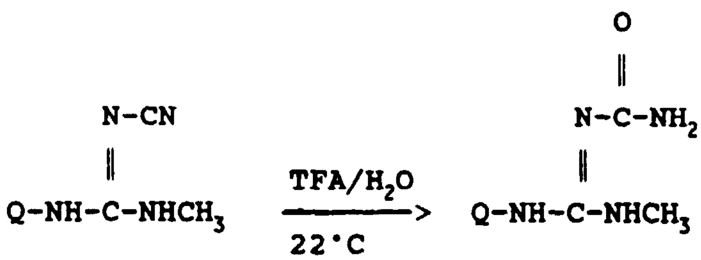


The hydrolyzed versions, referred to herein as including the N-g'-amido group, can also be synthesized directly by reacting phosgene derivates with moieties having a guanidino function.

If $HXR_2$ is an amino group of another peptide or protein, one will obtain a peptide-peptide dimer or peptide-protein dimer conjugated via the cyanoguanidine moiety. If $HXR_2$ is the N-terminal primary amino group or the side chain amino group of another amino acid in the same peptide, one will obtain a cyclic peptide (IV) linked via the cyanoguanidine moiety:

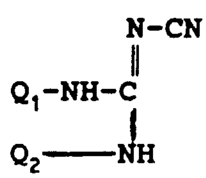

(IV)

wherein $Q_1$ and $Q_2$ represent the remainders of two amino acid residues in the same peptide. Cyclization via the cyanoguanidine derivative is preferably effected while a part of the peptidoresin, as opposed to subsequently cyclizing the linear peptide.

A special case arises when $-XR_2$ contains a second nucleophilic site and X has the general form: $M_1\text{-}(CH_q)_p\text{-}M_2$ or $M_1\text{-}(CH_2)_{p'}\text{-}M_2\text{-}(CH_2)_{p''}\text{-}M_3$, where $M_1$, $M_2$ and $M_3$ are individually NH, N, O or $CHR_3$, with p, p', p" being 0,1,2 or 3 and q being 1 or 2. Examples of such nucleophiles include $H_2NNH_2$, $CH_3HNNH_2$, $CH_3HNNHCH_3$, $H_2NOH$, and $H_2N\text{-}CH_2\text{-}CH_2OH$. In this case, the cyanoguanidine moiety that is formed can be converted into the corresponding heterocycle (V) which forms from the initial intermediate by reaction of the omega amino group with the cyano group such as:

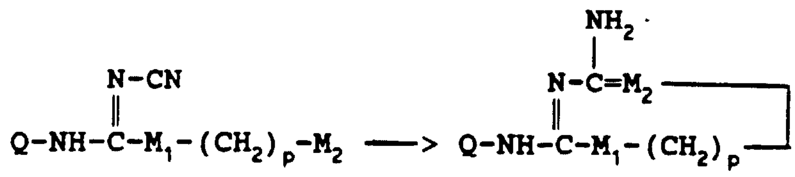

(V)

For example, where $-XR_2 = -HNNH_2$,

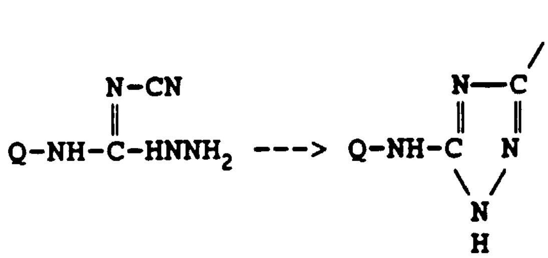

(1,2,4 triazole moiety)

Furthermore, where $-XR_2 = -CH_3NNHCH_3$

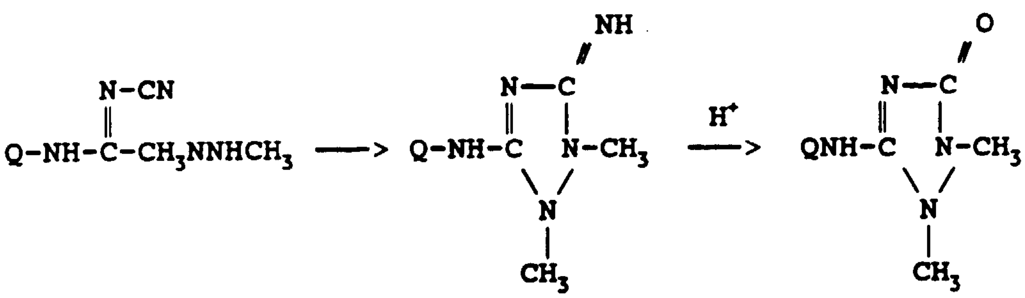


which may then undergo hydrolysis, as indicated above.

Where $XR_2$ contains a carboxylic acid group or the equivalent, particularly a carboxylic ester or carboxylic amide, a heterocyclic moiety, such as a saturated pyrimidine-like moiety (VI), is formed, by reaction of the carboxylic group with the secondary amino group ($R_1$), when $M_1$ is N, and similar 6-membered heterocyclic moieties are formed when $M_1$ is O or S. For example, $R_2$ may be $M_1$-$(CH_2)_p$-$M_2$ with $M_2$ = COOH, $COOCH_3$ or $CONH_2$ and p being an integer between 1 and 4. For instance in such a case where an aliphatic carboxylic acid group is present and p=2:

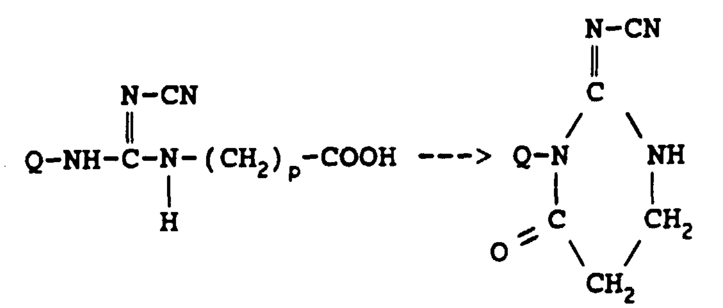

(VI) 3-peptide-2-(cyanoimino)-4 oxohexahydro-pyrimidino

If $R_2$ includes an ortho-substituted aromatic carboxylic acid, e.g. benzoic acid (q=1 and p=6), the corresponding quinazoline-like species (VII) is formed:

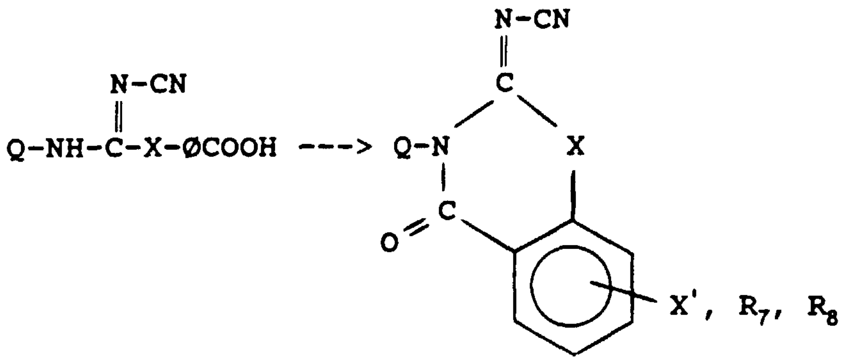

(VII)

Such benzoic acid may be further substituted, and such substitutions may in any of the other 4 ring positions, as shown, creating the corresponding substituted quinazoline-like moiety which is considered to be equivalent to the unsubstituted. X' may be H, Cl, Br, F, $NHCH_3$ or $SCH_3$, and $R_7$ and $R_8$ may be H, $CH_3$ or $CH_2CH_3$.

The molecules wherein X = $N_3$ and $R_2$ is des$R_2$ (i.e. deleted) are useful for photolabeling because of the activity of the -$N_3$ group and are formed by reacting the moiety (II) with sodium azide ($NaN_3$).

Peptides wherein Y is O (herein referred to as ureas) or S (referred to as thioureas) are prepared by the well known procedure in which the desired side chain amino group is treated with an appropriate isocyanate or thioisocyanate to obtain such ureas or thioureas.

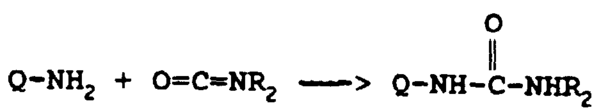

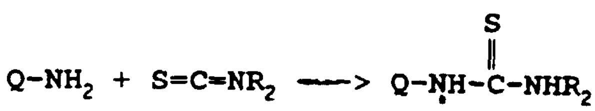

Peptides or amino acids wherein Y is CH-$NO_2$ (herein referred to as diaminonitroethylenes) are prepared by conversion of the corresponding urea to a carbodiimide:

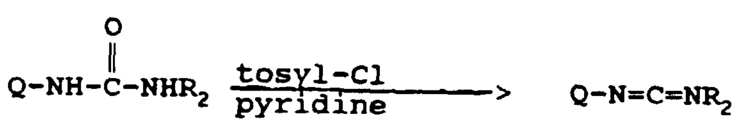


followed by treatment with nitromethane anion (prepared by the action of sodium hydride on nitromethane in dry DMF) as disclosed generally in F. Meimas, et al., Synthesis, 509-510 (1985):

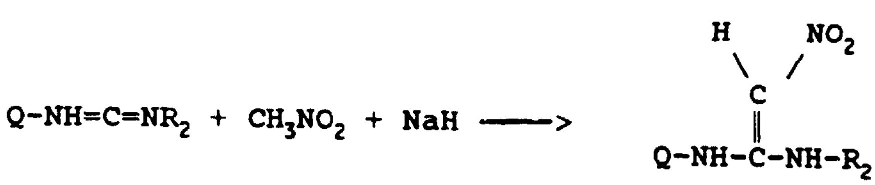

An alternative synthesis that may be used is as follows:

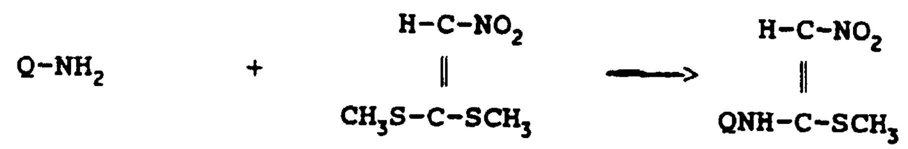

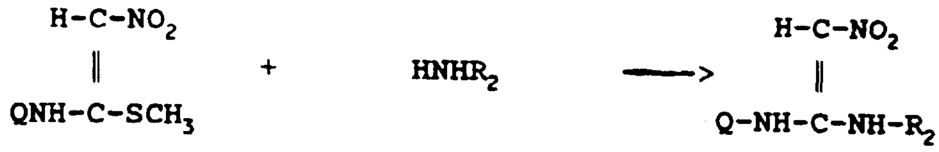

Generally, in accordance with the present invention, peptides are synthesized which are antagonists of GnRH, i.e., they strongly inhibit the secretion of gonadotropins by the pituitary gland of mammalians, including humans, and/ or inhibit the release of steroids by the gonads. These peptides are analogs of GnRH containing one or more unnatural amino acids of the formula U* in the 3-position, the 5-position and/or the 6-position. Preferably they are present only in the 5-and 6-positions. All the peptides contain at least one D-isomer, which may be the unnatural amino acid. When U* is in the 3- and/or 6-position, it is always in the form of a D-isomer; whereas when U* is in the 5-position, it is always in the form of an L-isomer. The antagonist should have a 1-position substitution, preferably β-(1-or 2-naphthyl)-D-alanine (hereinafter β-D-1NAL or β-D-2NAL) or dehydroPro, a 2-position substitution in the form of a modified D-Phe, preferably 4Cl or 4F, and a 3-position substitution, preferably in the form of substituted or unsubstituted D-Trp, D-3PAL, β-D-NAL or the residue of a D-isomer amino acid U*, most preferably D-3PAL. The 5-position may be occupied by (a) Tyr, (b) a halogenated or methylated Phe or Tyr, (c) Arg, (d) Lys in which the side chain amino group is acylated by 3-carboxypyridine (nicotinic acid) or by 2 or 4-carboxypyridine, i.e. Lys(cpd), preferably Lys(3cpd) which is also referred to as Lys(Nic), (e) His or (f) the residue of an L-isomer amino acid U*, preferably the latter. The antagonists preferably have a D-isomer U* but may have a substituted or acylated D-Lys in the 6-position. If Leu is not in the 7-position, it is preferably NML; however, it may be Nle, Phe, Nva, Met, Tyr, Trp or PAL (of which the Phe or Trp residue may be substituted). The antagonists may also have an optional substitution in the 8-position, which is preferably isopropyl Lys, i.e., ILys or Lys(Ipr) wherein the side chain amino group is substituted by isopropyl. If a substitution is made in the 10-position, it is preferably D-Ala. At least one residue of a D-isomer amino acid of the formula U* is most preferably present in each peptide of the invention.

Modified D-Phe in the 2-position provides increased antagonistic activity as a result of the specific modifications present in the benzene ring. Single substitutions for hydrogen in the ring are preferably made in the para- or 4-position, but might be in either the 2- or 3-position also; the substitutions are selected from chloro, fluoro, bromo, methyl, methoxy and nitro, with 4 chloro being most preferred. Dichloro substitutions are in the 2,4 or 3,4 positions in the ring. The α-carbon atom may also be methylated, e.g. ($C^aMe$/4Cl)Phe. The 1-position substituent is preferably modified so that its α-amino group contains an acyl group, such as formyl(For), acetyl(Ac), acrylyl(Acr), vinylacetyl (Vac) or benzoyl(Bz), with acetyl and acrylyl being preferred and with acetyl being most preferred. PAL and D-PAL represent the L- and D-isomers of pyridylalanine where the β-carbon of Ala is linked to the 2-, 3- or 4-position, preferably to the 3-position, on the pyridine ring. When β-D-NAL is present in the 1-position and $R_5$ is not Arg, a hydrophilic D-amino acid residue, such as $4NH_2$-D-Phe, 4-guanidino-D-Phe, D-His, D-Lys, D-Orn, D-Arg, D-Har(Homoarginine) or D-PAL is preferably present in the 6-position if U* is not present. When dehydroPro is present in the 1-position, D-PAL or a D-isomer of a lipophilic amino acid, such as D-Trp, D-Phe, For-D-Trp, $NO_2$-D-Trp, D-Leu, D-Ile, D-Nle, D-Tyr, D-Val, D-Ala, dialkyl Arg, dialkyl Har, D-Ser(OtBu), β-D-NAL or (imBzl)D-His is preferably in the 6-position, if U* is not present.

These GnRH analogs are very soluble at a pH just below physiological pH, i.e. at about 4.5 to about 6, and thus can be formulated and administered in concentrated form, greatly facilitating administration at a pH of about 5 to 7.4 which is presently preferred. These antagonists inhibit ovulation of female mammals when administered at low levels at proestrus and are also effective to cause resorption of fertilized eggs if administered shortly after conception. These antagonists are also effective for the contraceptive treatment of male mammals and the treatment of steroid-dependent tumors. Certain of these antagonists are surprisingly long-acting in their suppression of LH levels following administration, and certain have a particularly low side effect in respect of histamine release.

DETAILED DESCRIPTION OF THE PREFERRED EMBODIMENTS

As previously mentioned, the unnatural amino acids (which can be L- or D-isomers) are represented by the formula U*:

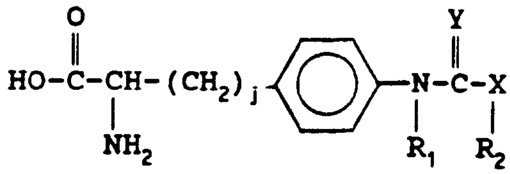

wherein W, X, Y, $R_1$ and $R_2$ are as defined previously, and there is at least one such residue (preferably a D-isomer) in each peptide of the invention.

More specifically, the GnRH antagonists of the present invention are represented by the following Formula ($F_1$) :

G-$AA_1$-(A)D-Phe-$AA_3$-Ser-$AA_5$-$AA_6$-$AA_7$-$AA_8$-Pro-$AA_{10}$ wherein G is hydrogen or an acyl group having 7 or less carbon atoms; $AA_1$ is dehydroPro, D-pGlu, (A)D-Phe, (B)D-Trp, Pro, or β-D-NAL; A is H, Cl, F, $NO_2$, $CH_3$, $OCH_3$, $C^{\alpha}$Me/4Cl, $Cl_2$ or Br; B is H, $NO_2$, $NH_2$, $OCH_3$, F, Cl, Br, $CH_3$, $N^{in}$For or $N^{in}$Ac; $AA_3$ is U*, D-PAL, β-D-NAL or (B)D-Trp; $AA_5$ is U*, Tyr, (C)Arg, Lys(cpd), Orn(cpd), Dbu(cpd), Dpr(cpd), (A)Phe, (3I)Tyr or His; $AA_6$ is U*, β-D-NAL, (B)D-Trp, (A')D-Phe, (D)D-Orn, (D)D-Lys, (D)D-Dbu, (D)D-Dpr, D-Har, D-Tyr, (E)D-His, D-PAL, (C)D-Arg or a suitable lipophilic D-isomer; A' is A, $NH_2$, $NHCH_3$ or gua; C is H or lower alkyl; D is G, cpd or an aryl group; E is H, imBzl or dinitrophenol; $AA_7$ is Nle, Leu, NML, (A)Phe, Met, Nva, Tyr, (B)Trp or PAL; $AA_8$ is (C')Arg, (C')Har or ILys; C' is H or di-lower alkyl; $AA_{10}$ is D-Ala-$NH_2$, Gly-$NH_2$, AzaGly-$NH_2$ or NH(R); R is lower alkyl, preferably $CH_2CH_3$; and U* is as defined above. When $AA_1$ is β-D-NAL and $AA_5$ is not Arg, then $AA_6$ is preferably U*, 4-$NH_2$-D-Phe, D-Lys, D-Orn, D-Har, D-His, 4-gua-D-Phe, D-PAL or D-Arg.

By dehydroPro is meant 3,4 dehydroproline, $C_5H_7O_2N$. By β-D-NAL is meant the D-isomer of alanine which is substituted by naphthyl on the β-carbon atom, i.e., also 3-D-NAL. Preferably β-D-2NAL is employed wherein the attachment to naphthalene is at the 2-position on the ring structure; however, β-D-1NAL may also be used. The preferred 1-position residues are β-D-NAL, substituted D-Phe and optionally substituted D-Trp. PAL represents alanine which is substituted by pyridyl on the β-carbon atom; preferably the linkage is to the 3-position on the pyridine ring. When substituted D-Trp is employed, single substitutions for hydrogen are preferably made in either the 5- or 6-position, which are selected from chloro, fluoro, bromo, methyl, amino, methoxy and nitro, with chloro, fluoro and nitro being preferred.

Alternatively, the indole nitrogen may be acylated, e.g. with formyl ($N^{in}$For- or 1For-) or with acetyl. D-3PAL, $N^{in}$For-D-Trp and 6$NO_2$-D-Trp are the preferred residues for the 3-position although D-Trp is also often used. When U* is not in the 5-position, it is preferably Tyr, Arg or Lys(cpd). By NML is meant $N^{\alpha}CH_3$-Leu. By Dbu is meant alpha, gamma diamino butyric acid, and by Dpr is meant α,β diamino propionic acid. By Aph is meant 4$NH_2$Phe. By Hap is meant 4-amino-homophenylalanine (where j is 2); by Hhp is meant 4-amino-homohomophenylalanine (where j is 3). When dehydroPro is present in the 1-position, Tyr or U* is preferably present in the 5-position and a lipophilic residue is in the 6-position. By 4-gua-D-Phe is meant a residue of D-Phe having guanidine substituted in the para-position. By AzaGly-$NH_2$ is meant $NHNHCONH_2$. The guanidino group of an Arg residue in the 5- or 6-position may be substituted by lower alkyl, i.e. 1 to 4 carbon atoms, e.g., propyl(Pr). When D-Lys, D-Dbu, D-Dpr or D-Orn is present in the 6-position, its side-chain-amino group may be acylated by an acyl group which may be aliphatic, heterocyclic or aromatic, e.g. nicotinic acid, or may be substituted by an aryl group having not more than 1 phenyl ring. When U* is not present in the 6-position, it is preferably D-PAL or D-Lys(cpd) and most preferably D-3PAL. The 7-position residue is preferably Leu, NML, Nle or Phe and most preferably Leu or NML. The 8-position residue is preferably either Arg or ILys, but most preferably ILys.

One preferred subgenus of GnRH antagonists has the following formula:

Ac-$AA_1$-(A)D-Phe-$AA_3$-Ser-$AA_5$-$AA_6$-$AA_7$-$AA_8$-Pro-$AA_{10}$ wherein $AA_1$ is β-D-NAL, (A)D-Phe, (B)D-Trp or dehydro Pro; A is H, 4Cl,4F, 4$NO_2$, 4$CH_3$, 4$OCH_3$, $C^{\alpha}$e/4Cl, 2,4$Cl_2$ or 4Br; B is H, 6$NO_2$, 6$NH_2$, 6$OCH_3$, 6F, 6Cl, 6Br, 6$CH_3$, $N^{in}$For or $N^{in}$Ac;

$AA_3$ is U*, D-PAL, β-D-NAL or (B)D-Trp; $AA_5$ is U*, Lys(cpd) or Tyr; $AA_6$ is U*, β-D-NAL, 4$NH_2$D-Phe, (B)D-Trp, D-Lys (cpd), D-PAL or D-Arg; $AA_7$ is Nle, Leu, NML or Phe; is ILys, or Arg; $AA_{10}$ is D-Ala-$NH_2$, Gly-$NH_2$, $NHNHCONH_2$ or NH(R); R is lower alkyl; and U* is either

(a)

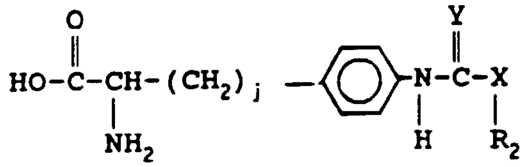

where j is 1,2 or 3; X is NH or O; Y is N-CN or N-CONHR$_9$ where R$_9$ is H or lower alkyl; R$_2$ is lower alkyl, cyclohexyl, phenyl, pyridyl, methyl pyridyl or histaminyl; or

(b)

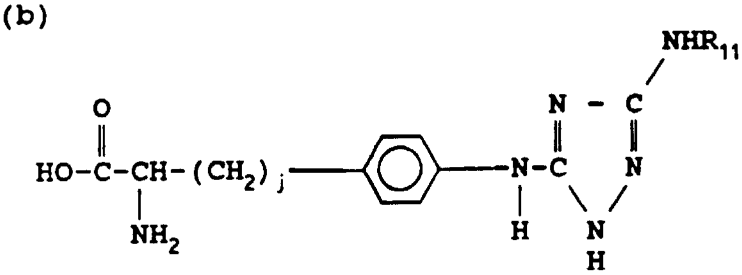

where j is 1 or 2 and R$_{11}$ is H or an acyl radical having 1 to 6 carbon atoms; provided, however, that at least one of AA$_3$, AA$_5$ and AA$_6$ is U*; preferably AA$_5$ and AA$_6$ are U*.

Another preferred subgenus of GnRH antagonists has the following formula:

Ac-AA$_1$-(A)D-Phe-U*-Ser-AA$_5$-AA$_6$-AA$_7$-AA$_8$-Pro-AA$_{10}$ wherein AA$_1$ is (A)D-Phe or β-D-NAL; A is 4Cl,4F or 4NO$_2$; B is H, 6NO$_2$ or N$^{in}$For; AA$_5$ is Lys(cpd) or Tyr; AA$_6$ is β-D-NAL, 4NH$_2$D-Phe, (B)D-Trp, D-Lys(cpd), D-PAL or D-Arg; AA$_7$ is Nle,

Leu, NML or Phe; AA$_8$ is ILys or Arg; AA$_{10}$ is D-Ala-NH$_2$, Gly-NH$_2$, NHNHCONH$_2$ or NH(R); R is lower alkyl; and U* is either

(a)

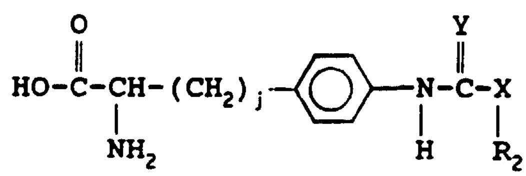

where j is 1, 2 or 3 (preferably 1); X is NH or O; Y is N-CN or N-CONHR$_9$ where R$_9$ is H or lower alkyl (C$_1$-C$_3$), preferably H; R$_2$ is lower alkyl (C$_1$-C$_6$), cyclohexyl, phenyl, pyridyl, methyl pyridyl or histaminyl; or

(b)

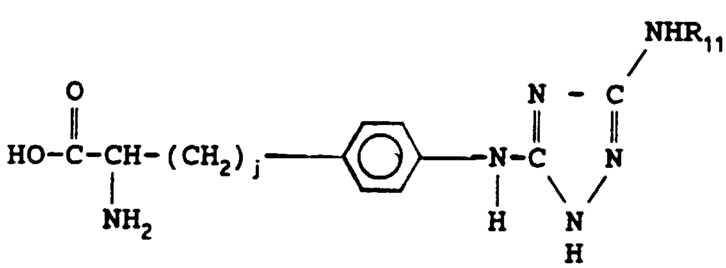

where j is 1 or 2 and $R_{11}$ is H or an acyl radical having 1 to 3 carbon atoms, preferably acetyl.

Yet another preferred subgenus of GnRH antagonists has the following formula: Ac-β-D-2NAL-(A) D-Phe-D-3PAL-Ser-$AA_5$-$AA_6$-$AA_7$-$AA_8$-Pro-$AA_{10}$ wherein A is 4Cl or 4F; $AA_5$ is U*, Lys(cpd) or Tyr; $AA_6$ is U*, β-D-NAL, D-Lys(cpd), D-3PAL or D-Arg; $AA_7$ is Leu or NML; $AA_8$ is ILys or Arg; $AA_{10}$ is D-Ala-$NH_2$ or Gly-$NH_2$; and U* is either

(a)

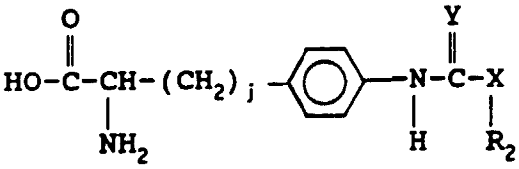

where j is 1,2 or 3 (preferably 1); X is NH or O; Y is N-CN or N-$CONHR_9$ where $R_9$ is H or lower alkyl ($C_1$-$C_3$), preferably H; $R_2$ is lower alkyl ($C_1$-$C_6$), cyclohexyl, phenyl, pyridyl or methyl pyridyl; or

(b)

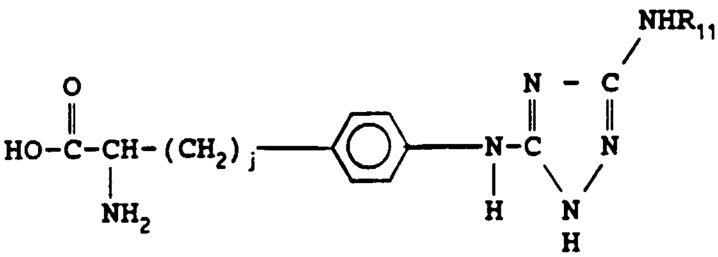

where j is 1 or 2 and $R_{11}$ is H or an acyl radical having 1 to 3 carbon atoms, preferably acetyl; provided that at least one and preferably both of $AA_5$ and $AA_6$ are U*.

The peptides of the present invention can be synthesized by classical solution synthesis, but are preferably synthesized by a solid phase technique. A chloromethylated resin or a hydroxymethylated resin may be used; however, a methylbenzhydrylamine(MBHA) resin, a benzhydrylamine (BHA) resin or some other suitable resin known in the art which directly provides a C-terminal amide or substituted amide upon cleavage is preferably employed when such a C-terminus is desired. For example, peptides having a substituted amide at the C-terminus are preferably synthesized using an N-alkylamino methyl resin as taught in United States Patent No. 4,569,967, issued February 11, 1986. Solid phase synthesis is conducted in a manner to stepwise add amino acids in the chain in the manner set forth in detail in the U.S. Patent No. 4,211,693. Side-chain protecting groups, as are well known in the art, are preferably included as a part of any amino acid which has a particularly reactive side chain and optionally in the case of others, such as Trp, which amino acids are to be coupled in the chain being built upon the resin. Such synthesis provides the fully protected intermediate peptidoresin.

Chemical intermediates made generally in accordance with the invention may be represented by the formula: $X^1$-$AA_1$-$AA_2$ ($X^5$) -$U_3$-Ser($X^3$)-$U_5$-$U_6$-$AA_7$($X^2$ or $X^7$)-$AA_8$($X^5$ or $X^6$)-Pro-$X^8$ wherein: $U_3$ is either U' or $AA_3$($X^2$) ; $U_5$ is either U' or $AA_5$($X^4$ or $X^5$) ; $U_6$ is either U' or $AA_6$($X^4$ or $X^5$ or $X^6$) ; U' is either Aph($X^a$), Hap($X^a$) or Hhp($X^a$); $X^1$ is an α-amino protecting group of the type known to be useful in the art in the stepwise synthesis of polypeptides and when G in the desired peptide composition is a particular acyl group, that group may be used as the protecting group. Among the classes of α-amino protecting groups covered by $X^1$ are (1) acyl-type protecting groups, such as formyl(For), trifluoroacetyl, phthalyl, p-toluene-sulfonyl(Tos), benzoyl(Bz), benzenesulfonyl, dithiasuccinoyl(Dts) o-nitrophenylsulfenyl (Nps), tritylsulfenyl, o-nitrophenoxyacetyl, acrylyl(Acr), chloroacetyl, acetyl(Ac) and γ-chlorobutyryl; (2) aromatic urethan-type protecting groups, e.g., benzyloxycarbonyl (Z), fluorenylmethyloxycarbonyl (Fmoc) and substituted benzyloxycarbonyl, such as p-chlorobenzyloxy-carbonyl (ClZ), p-nitrobenzyloxycarbonyl, p-bromobenzyloxycarbonyl and p-methoxybenzyloxycarbonyl; (3) aliphatic urethan protecting groups, such as tertbutyloxycarbonyl(Boc), diisopropylmethoxycarbonyl, isopropyloxycarbonyl, ethoxycarbonyl and allyloxycarbonyl; (4) cycloalkyl urethan-type protecting groups, such as cyclopentyloxycarbonyl, adamantyloxycarbonyl and cyclohexyloxycarbonyl; (5) thiourethan-type protecting groups, such as phenylthiocarbonyl; (6) alkyl-type protecting groups, such as allyl(Aly), triphenylmethyl(trityl)

and benzyl(Bzl); (7) trialkylsilane groups, such as trimethylsilane. The preferred α-amino protecting group is Boc when X is hydrogen.

$X^2$ is hydrogen or a protecting group for the indole nitrogen of Trp, such as Bz, Ac or For. In many syntheses there is no need to protect Trp, and such protection is not used if acylated D-Trp is present elsewhere in the peptide.

$X^3$ is a protecting group for the hydroxyl side chain of Ser or Thr, e.g. Ac, Bz, trityl, DCB or benzyl ether(Bzl) and is preferably Bzl.

$X^4$ is hydrogen or a protecting group for the phenolic hydroxyl group of Tyr selected from the group consisting of tetrahydropyranyl, tert-butyl, trityl, benzyl, Z, 2-bromobenzyloxycarbonyl(2BrZ) and 2,6-dichlorobenzyl(DCB). 2BrZ is preferred.

$X^5$ is a protecting group for a side chain guanidino group, such as that in Arg or Har, or for the imidazole group of His, such as nitro, Tos, trityl, adamantyloxycarbonyl, Z and 2,4-dinitrophenol(Dnp), or $X^5$ may be hydrogen, which means there is no protection on the side chain group atoms. Tos is generally preferred.

$X^6$ is a protecting group for an amino side chain group, primary or secondary amino, such as Z or 2ClZ; $X^a$ is a subclass of $X^6$ comprising such protecting groups that can be removed without removing other side chain protecting groups so as to allow the omega-amino group to thereafter take part in the reactions to build the unnatural amino-acid residue. Preferably a base-labile group, such as Fmoc, methylsulfonylethyloxycarbonyl(Msc) or trifluoroacetyl(Tfa), is used; however, it may also be possible to use a hydrazine-labile group such as phthaloyl,

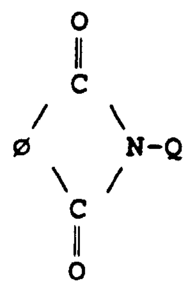

or a thiolabile group such as Nps or Dts.

$X^7$ is hydrogen or a protecting group for Met, such as oxygen; Met is generally left unprotected.

$X^8$ may be Gly-NH-[resin support], D-Ala-NH- [resin support] or N(A)-[resin support]; $X^8$ may also be an amide either of Gly or of D-Ala or a substituted amide attached directly to Pro or $NHNHCONH_2$.

The criterion for selecting side chain protecting groups for $X^2$-$X^7$ is that the protecting group should be stable to the reagent under the reaction conditions selected for removing the α-amino protecting group (preferably Boc) at each step of the synthesis. Protecting groups generally should not be split off under coupling conditions but should be removable upon completion of the synthesis of the desired amino acid sequence under reaction conditions that will not alter the peptide chain.

When the $X^8$ group is Gly-NH-[resin support] or D-Ala-NH-[resin support], an amide bond connects Gly or D-Ala to a BHA resin or to a MBHA resin. When the $X^8$ group is N(A)-[resin support], a substituted amide bond connects Pro to an N-alkylaminomethyl (NAAM) resin. When $X^8$ is AzaGly-$NH_2$, the peptide is preferably made by classical solution synthesis, as disclosed in U.S. Patent No. 4,234,571.

When G is acetyl, for example, in the final formula, it may be possible to employ it as the X1 protecting group for the α-amino group of β-D-NAL or whatever amino acid is used in the 1-position by adding it before coupling this last amino acid to the peptide chain. However, a reaction is preferably carried out with the peptide on the resin (after deblocking the α-amino group while the side-chain groups remain protected), e.g. by reacting with acetic acid in the presence of diisopropyl or dicyclohexyl carbodiimide (DIC or DCC) or preferably with acetic anhydride or by another suitable reaction as known in the art.

Thus, the invention also provides a method for making a peptide, said peptide having the formula:

G-$AA_1$-$AA_2$-$AA_3$-Ser-$AA_5$-$AA_6$-$AA_7$-$AA_8$-Pro-$AA_{10}$, wherein at least one of $AA_3$, $AA_5$ and $AA_6$ is U* and the symbols are as set forth hereinbefore, which method comprises (a) forming an intermediate peptide having the formula: $X^1$-$AA_1$-$AA_2$($X^5$)-$U_3$-Ser($X_3$)-$U_5$-$U_6$-$AA_7$($X^2$ or $X^7$)-$AA_8$ ($X^5$ or $X^6$)-Pro-$X^8$ wherein: $U_3$ is either U' or $AA_3$($X^2$) ; $U_5$ is either U' or $AA_5$($X^4$ or $X^5$) ; $U_6$ is either U' or $AA_6$($X^4$ or $X^5$ or $X^6$) ; U' is either Aph($X^a$), Hap($X^a$) or Hhp($X^a$); $X^1$ is hydrogen or an α-amino protecting group; $X^2$ is hydrogen or a protecting group for an indole nitrogen; $X^3$ is a protecting group for a hydroxyl group of Ser or Thr; $X^4$ is hydrogen or a protecting group for a phenolic hydroxyl group of Tyr; $X^5$ is either hydrogen or a protecting group for a guanidino or imidazole side chain; $X^6$ is a protecting group for a primary amino side chain of which $X^a$ is a subgroup that is removable without removing other protecting groups; $X^7$ is hydrogen

or a protecting group for Met; $X^8$ is Gly-NH-[resin support], D-Ala-NH-[resin support], N(A)-[resin support], an amide either of Gly or of D-Ala or a substituted amide attached directly to Pro or $NHNHCONH_2$; provided however that at least one of $U_3$, $U_5$ and $U_6$ is either Aph($X^a$), Hap($X^a$), or Hhp($X^a$) ; (b) removing at least one $X^a$ to deprotect a side chain primary amino group of at least one amino acid residue of said intermediate peptide; (c) reacting said deprotected side chain primary amino group to build said residue into one having the formula U*; and (d) splitting off any remaining groups $X^1$ to $X^7$ and/or cleaving from any resin support included in $X^8$.

Purification of the peptide is effected by ion exchange chromatography on a CMC column, followed by partition chromatography using the elution system: n-butanol;0.1N acetic acid (1:1 volume ratio) on a column packed with Sephadex G-25, or by using HPLC, as known in the art and specifically set forth in J. Rivier, et al. J. Chromatography, 288 (1984) 303-328.

The antagonists of the invention are effective at levels of less than 100 micrograms per kilogram of body weight, when administered subcutaneously at about noon on the day of proestrus, to prevent ovulation in female rats. For prolonged suppression of ovulation, it may be necessary to use dosage levels in the range of from about 0.1 to about 2.5 milligrams per kilogram of body weight. These analogs are particularly soluble at physiologically acceptable pHs and thus can be prepared as relatively concentrated solutions for administration. The antagonists are also effective to arrest spermatogenesis when administered to male mammals on a regular basis and can thus be used as contraceptives. Since these compounds will reduce testosterone levels (an undesired consequence in the normal, sexually active male), it may be reasonable to administer replacement dosages of testosterone along with the GnRH antagonist. These antagonists can also be used to regulate the production of gonadotropins and sex steroids for other purposes as indicated hereinbefore.

In the following formulas, the U* residues are defined in terms of the original amino acid residue having a side chain amino group plus the modification in question which is set forth in the accompanying parentheses. Preferably, the original residue Aph or Hap or Hhp, or the D-isomer form thereof, is incorporated in the main peptide chain and is modified while a part of the peptide chain that is still attached to the resin to form the desired residue of the amino acid U*. However, as indicated hereinbefore, the suitably protected unnatural amino acid U* can be added as a part of the usual chain elongation process.

With respect to ultimate modified side chain omega-amino group, the following abbreviations are used:

act=acetyl aminotriazole
bcg=aminobutyl cyanoguanidino
bzcg=aminobenzyl cyanoguanidino
bur=N-g-amido, N-g'-butylguanidino
chcg=aminocyclohexyl cyanoguanidino
ecg=aminoethyl cyanoguanidino
icg=aminoisopropyl cyanoguanidino
hcg=aminohexyl cyanoguanidino
hicg=histaminyl cyanoguanidino (ethylimidazole)
mcg=aminomethyl cyanoguanidino
ncg=aminoethyl(1 or 2)naphthyl cyanoguanidino
mncg=aminomethyl(1 or 2)naphthyl cyanoguanidino
Ocg=O-phenyl cyanoguanidino
pcg=aminopropyl cyanoguanidino
Sbcg=thiobutyl cyanoguanidino
tcg=3-amino 1,2,4 triazole
trcg=indole ethylamino cyanoguanidino(tryptamino cyanoguanidino)
mpcg= aminomethyl pyridyl cyanoguanidino (number indicates position of aminomethyl group on pyridyl ring)

EXAMPLE 1

Peptides as indicated in TABLE 1 having the formula: Ac-β-D-2NAL-(4Cl) D-Phe-D-3PAL-Ser-$AA_5$-$AA_6$-Leu-$AA_8$-Pro-D-Ala-$NH_2$ are prepared by the solid-phase procedure described generally hereinbefore.

TABLE 1

| | $AA_5$ | $AA_6$ | $AA_8$ |
|---|---|---|---|
| 103A | Aph(tcg) | D-Aph(tcg) | ILys |
| 104A | Aph(bcg) | D-Aph(bcg) | ILys |

TABLE 1 (continued)

| | $AA_5$ | $AA_6$ | $AA_8$ |
|---|---|---|---|
| 105B | Hap(tcg) | D-Lys(Nic) | ILys |
| 106C | Hhp(tcg) | D-Hhp(tcg) | ILys |
| 107A | Aph(2ncg) | D-Aph(2ncg) | ILys |
| 108A | Hhp(bcg) | D-Hhp(bcg) | ILys |
| 109A | Hap(tcg) | D-Hap(tcg) | Arg |
| 110A | Aph(icg) | D-Aph(icg) | ILys |

As a working example, a solid phase synthesis is set forth hereinafter of Peptide No. 103A above, which is referred to as [Ac-β-D-2NAL$^1$, (4Cl)D-Phe$^2$, D-3PAL$^3$, Aph(tcg)$^5$, D-Aph(tcg)$^6$, ILys$^8$, D-Ala$^{10}$]-GnRH. This peptide has the following formula:
[Ac-β-D-2NAL-(4Cl)D-Phe-D-3PAL-Ser-Aph(3-amino 1,2,4 triazole)-D-Aph(3-amino 1,2,4 triazole)-Leu-Lys(isopropyl)-Pro-D-Ala-$NH_2$.

An MBHA resin is used, and Boc-protected D-Ala is coupled to the resin over a 2-hour period in $CH_2Cl_2$ using a 3-fold excess of Boc derivative and DCC as an activating reagent. The D-Ala residue attaches to the MBHA residue by an amide bond.

Following the coupling of each amino acid residue, washing, deblocking and coupling of the next amino acid residue is carried out in accordance with the following schedule using an automated machine and beginning with about 5 grams of resin:

| STEP | REAGENTS AND OPERATIONS | MIX TIMES MIN. |
|---|---|---|
| 1 | $CH_2Cl_2$ wash-80 ml. (2 times) | 3 |
| 2 | Methanol(MeOH) wash-30 ml. (2 times) | 3 |
| 3 | $CH_2Cl_2$ wash-80 ml. (3 times) | 3 |
| 4 | 50 percent TFA plus 5 percent 1,2-ethanedithiol in $CH_2Cl_2$-70 ml. (2 times) | 10 |
| 5 | Isopropyl alcohol + 1% ethanedithiol wash-80 ml. (2 times) | 3 |
| 6 | TEA 12.5 percent in $CH_2Cl_2$-70 ml. (2 times) | 5 |
| 7 | MeOH wash-40 ml. (2 times) | 2 |
| 8 | $CH_2Cl_2$ wash-80 ml. (3 times) | 3 |
| 9 | Boc-amino acid (10 mmoles) in 30 ml. of either dimethylformamide(DMF) or $CH_2Cl_2$, depending upon the solubility of the particular protected amino acid, (1 time) plus DIC or DCC (10 mmoles) in $CH_2Cl_2$ | 30-300 |
| 10 | MeoH wash-40 ml. (2 times) | 3 |
| 11 | Triethylamine(TEA) 12.5 percent in $CH_2Cl_2$-70 ml. (1 time) | 3 |

After step 3, an aliquot may be taken for a ninhydrin test as well known in the art: if the test is negative, proceed to step 4 for removal of BOC-group prior to coupling of the next amino acid; if the test is positive or slightly positive, repeat steps 9 through 11.

The above schedule is used for coupling of each of the amino acids of the peptide of the invention after the first amino acid has been attached. $N^{\alpha}$Hoc protection is used for each of the remaining amino acids throughout the synthesis. $N^{\alpha}$Boc-β-D-2NAL is prepared by a method known in the art, e.g. as described in detail in U.S. Patent No. 4,234,571, issued November 18, 1980 or commercially available from SyntheTech, Oregon, U.S.A. The side chain primary amino groups of Aph in the 5-position and of D-Aph in the 6-position are protected by Fmoc. Bzl(benzyl ether) is used as a side chain protecting group for the hydroxyl group of Ser. Boc-Lys(Ipr,Z) is used for the 8-position. After deblocking the α-amino group at the N-terminus using trifluoroacetic acid (TFA), acetylation is achieved using a large excess of acetic anhydride in dichloromethane.

Following completion of the assembly of the peptide and acetylation of the N-terminus, the following intermediate is present: Ac-β-D-2NAL-(4Cl)D-Phe-D-3PAL-Ser(Bzl)-Aph(Fmoc)-D-Aph(Fmoc)-Leu-Lys(Ipr,Z)-Pro-D-Ala-NH-[MBHA resin support]. The unnatural amino acids in the 5- and 6-positions are formed by simultaneously carrying out the following reactions with the deprotected side chains of the Aph residues. The Fmoc protecting group is removed from both by treatment of the peptidoresin with 20 percent piperidine in DMF for 5 minutes, then washing with DMF, then treatment with more piperidine/DMF for 20 minutes. After washing the resin with DMF, $CH_3OH$, $CH_2Cl_2$, and finally DMF, the newly freed amino group is treated with a large excess (>10 fold) of diphenyl cyanocarbonimidate(PCI) in

DMF. Thereafter, the peptide is then subjected to the standard wash and then treated with hydrazine, dissolved in DMF, for 24 hours at about 22°C. to complete the formation of the cyanoguanidino moiety; this step is preferably repeated. The cyanoguanidino moieties that are formed spontaneously convert to the corresponding heterocycle, i.e. 3-amino, 1,2,4 triazole.

The cleavage of the peptide from the resin and deprotection of the Ser and the Lys side chains takes place very readily at 0°C. with HF. Anisole is added as a scavenger prior to HF treatment. After the removal of HF under vacuum, the resin is extracted with 50% acetic acid, and the washings are lyophilized to provide a crude peptide powder.

Purification of the peptide is then effected by high performance liquid chromatography (HPLC), as known in the art and specifically set forth in J. Rivier, et al. J. Chromatography, 288 (1984) 303-328.

The peptide is judged to be homogeneous using capillary zone electrophoresis (CZE), as well as by using reversed-phase high pressure liquid chromatography and an aqueous triethylammonium phosphate solution plus acetonitrile. Amino acid analysis of the resultant, purified peptide is consistent with the formula for the prepared structure, showing substantially integer-values for each amino acid in the chain; mass spectral analysis is also consistent. The optical rotation is measured on a photoelectric polarimeter as

$[\alpha]_D^{20}$ = -33 ±1.0(c=1, 50% acetic acid).

The other peptides in Table 1 are similarly synthesized and purified. The peptides are assayed in vivo which determines their effectiveness to prevent ovulation in female rats. In this test, a specified number of mature female Sprague-Dawley rats, e.g. five to ten, each having a body weight from 225 to 250 grams, are injected with a specified microgram dosage of peptide in either saline, bacteriostatic water, polyethylene glycol, corn oil or mixtures of the above with ethanol at about noon on the day of proestrus. Proestrus is the afternoon of ovulation. A separate female rat group is used as a control to which the peptide is not administered. Each of the control female rats ovulates on the evening of proestrus; of the rats treated, the number of them which ovulate is recorded. In vivo testing of peptide 103A shows that at a dosage of 2.5 micrograms, 0 out of 8 rats treated ovulate, and at a dosage of 1 microgram, 0 out of 7 rats ovulate. The peptide shows some activity at even 1/2 microgram where only 8 out of 18 rats ovulate. Peptide 104A is tested at 5 micrograms and shows that only 6 out of 11 rats ovulate.

In addition to the working synthesis set forth above, Peptide 103A is also synthesized with glycinamide at the C-terminus instead of D-Ala-$NH_2$; biological testing in vivo shows it is only slightly less biologically potent than Peptide 103A.

Peptide 103A was also synthesized with the N-ethylamide at the C-terminus instead of D-Ala-$NH_2$; biological testing in vivo showed it exhibited just slightly less biological potency than for Peptide 103A. In addition, Peptide 104A is also synthesized with the glycinamide at the C-terminus instead of D-Ala-$NH_2$; biological testing in vivo shows somewhat less biological potency than Peptide 104A.

All peptides listed in Table 1 are considered effective to block GnRH-induced LH secretion in vitro at some reasonable concentration. All of the peptides are considered to be effective to prevent ovulation of female mammals at low dosages.

In addition to the above-mentioned in vivo testing, to determine effectiveness to prevent ovulation in female rats, groups of about 6 adult castrate male Sprague-Dawley rats, each having a body weight from 225 to 250 grams, are each injected IV with a 50 microgram dosage of either Peptide No. 103A or a standard antagonist referred to as the Nal-Glu antagonist (see J. Clin. Edno. Metab 71, 4, 881-888 (1990)) in either corn oil or a phosphate buffer in the presence of BSA (bovine serum albumin). A separate rat group is used as a control, to which only the carrier is administered. Each of the control rats and the rats being treated is monitored for LH levels in the bloodstream at about 3 hours, 24 hours, 36 hours and 48 hours following this single bolus injection. Peptide No. 103A is surprisingly effective to suppress the level of circulating LH in the bloodstream of male rats, at levels very substantially below that of the control and, after 12 hours, below that of the rats treated with the Nal-Glu antagonist. This very long-acting duration of biological effect was truly surprising.

EXAMPLE 2

Peptides as indicated in TABLE 2 having the formula:
Ac-β-D-2NAL-(4Cl)D-Phe-D-3PAL-Ser-$AA_5$-$AA_6$-$AA_7$-ILys-Pro-$AA_{10}$ are prepared by the solid-phase procedure referred to above.

TABLE 2

| | $AA_5$ | $AA_6$ | $AA_7$ | $AA_{10}$ |
|---|---|---|---|---|
| 111A | Aph(tcg) | D-Aph(tcg) | NML | D-Ala-$NH_2$ |
| 112A | " (bcg) | " (bcg) | " | " |

TABLE 2 (continued)

| | $AA_5$ | $AA_6$ | $AA_7$ | $AA_{10}$ |
|---|---|---|---|---|
| 113B | " (bcg) | " (bcg) | Leu | Gly-$NH_2$ |
| 114B | " (tcg) | " (tcg) | " | $NHCH_2CH_3$ |
| 115A | " (tcg) | " (tcg) | NML | " |
| 116A | " (tcg) | " (tcg) | Leu | Gly-$NH_2$ |
| 117B | " (2mpcg) | " (2mpcg) | NML | D-Ala-$NH_2$ |
| 118A | " (4mpcg) | " (4mpcg) | " | " |
| 119A | " (icg) | " (icg) | " | " |
| 120B | " (ecg) | " (ecg) | " | " |
| 121A | " (3mpcg) | β-D-2NAL | " | " |
| 122B | " (tcg) | " | Leu | " |
| 123A | Lys(Nic) | D-Aph(tcg) | " | " |
| 124A | Hap(bcg) | β-D-2NAL | " | " |
| 125C | Hhp(bcg) | D-3PAL | " | " |
| 126A | Tyr | D-Aph(tcg) | " | " |

All peptides listed in Table 2 are considered effective to block GnRH-induced LH secretion <u>in vitro</u> at some reasonable concentration. All of the peptides are considered to be effective to prevent ovulation of female mammals at low dosages.

<u>EXAMPLE 3</u>

Peptides as indicated in TABLE 3 having the formula: Ac-β-D-2NAL-(4Cl)D-Phe-$AA_3$-Ser-$AA_5$-$AA_6$-Leu-$AA_8$-pro-D-Ala-$NH_2$ are prepared by the solid-phase procedure referred to above.

TABLE 3

| | $AA_3$ | $AA_5$ | $AA_6$ | $AA_8$ |
|---|---|---|---|---|
| 127A | D-Aph(tcg) | Tyr | D-3PAL | ILys |
| 128A | " (icg) | " | " | " |
| 129A | " (bcg) | " | " | " |
| 130A | " (tcg) | " | " | Arg |
| 131A | " (bcg) | Arg | β-D-2NAL | " |
| 132A | " (2mpcg) | " | " | " |
| 133A | " (tcg) | " | " | " |
| 134A | " (bcg) | Tyr | D-Arg | ILys |
| 135A | " (tcg) | " | " | " |
| 136A | D-Hap(tcg) | " | D-3PAL | Arg |
| 137A | " (bcg) | " | β-D-2NAL | ILys |
| 138A | D-Hhp(bcg) | " | D-3PAL | " |
| 139A | " (2mpcg) | " | " | " |
| 140A | D-Aph(2mpcg) | Arg | β-D-2NAL | Arg |
| 141A | D-Hhp(bcg) | Tyr | D-Arg | " |
| 142A | D-Hap(bcg) | Hap(bcg) | D-Hap(bcg) | ILys |
| 143A | D-Aph(bcg) | Tyr | D-Aph(bcg) | " |
| 144A | D-3PAL | Aph(bur) | β-D-2NAL | Arg |
| 145A | D-Aph(bur) | " " | " | " |
| 146A | " (2mpcg) | Tyr | " | ILys |
| 147A | " (tcg) | Tyr | D-Aph(tcg) | " |
| 148A | " (tcg) | Aph(tcg) | D-3PAL | " |

All peptides listed in Table 3 are considered effective to block GnRH-induced LH secretion <u>in vitro</u> at some

reasonable concentration. All of the peptides are considered to be effective to prevent ovulation of female mammals at low dosages.

EXAMPLE 4

Peptides as indicated in TABLE 4 having the formula: Ac-dehydroPro-(A)D-Phe-$AA_3$-Ser-$AA_5$-D-Aph(tcg)-Leu-ILys-Pro-D-Ala-$NH_2$ are prepared by the solid-phase procedure referred to above.

TABLE 4

| | A | $AA_3$ | $AA_5$ |
|---|---|---|---|
| 149 | 4Cl | β-D-2NAL | Tyr |
| 150 | " | " | " |
| 151 | 4F | (1For)D-Trp | (2F)Phe |
| 152 | " | " | Tyr |
| 153 | " | " | $(2NO_2)$Phe |
| 154 | " | (1Ac)D-Trp | $(2CH_3)$Phe |
| 155 | 4Br | " | Tyr |
| 156 | " | " | (2Br)Phe |
| 157 | H | D-Trp | (2Cl)Phe |
| 158 | $4NO_2$ | $(5CH_3)$D-Trp | $(3CH_3)$Phe |
| 159 | " | (5F)D-Trp | His |
| 160 | $2,4Cl_2$ | (5Cl)D-Trp | (3F)Phe |
| 161 | " | $(6NO_2)$D-Trp | (3Br)Phe |
| 162 | $C^{\alpha}$ Me/4Cl | $(5OCH_3)$D-Trp | (3I)Tyr |
| 163 | $3,4Cl_2$ | $(5NH_2)$D-Trp | (3Cl)Phe |

All peptides listed in Table 4 are considered effective to block GnRH-induced LH secretion in vitro at some reasonable concentration. All of the peptides are considered to be effective to prevent ovulation of female mammals at low dosages.

EXAMPLE 5

Peptides as indicated in TABLE 5 having the formula:
G-β-D-2NAL-(4Cl)D-Phe-D-3PAL-Ser-Aph(tcg) -$AA_6$-Leu-ILys-Pro-$AA_{10}$ are prepared by the solid-phase procedure referred to above.

TABLE 5

| | G | $AA_6$ | $AA_{10}$ |
|---|---|---|---|
| 164 | Acr | D-Arg | D-Ala-$NH_2$ |
| 165 | Ac | D-Arg | " |
| 166 | Acr | D-Aph(tcg) | " |
| 167 | For | D-Tyr | Gly-$NH_2$ |
| 168 | Bz | (Et)D-Arg | " |
| 169 | Ac | D-Lys | " |
| 170 | Vac | D-Har | " |
| 171 | Acr | (4gua)D-Phe | AzaGly-$NH_2$ |
| 172 | Ac | D-Orn | D-Ala-$NH_2$ |
| 173 | Acr | D-His | " |
| 174 | Ac | (Bu)D-Arg | " |
| 175 | " | (Bz)D-orn | " |
| 176 | Vac | $(4NH_2)$D-Phe | " |
| 177 | Bz | (Ac)D-Lys | AzaGly-$NH_2$ |

All peptides listed in Table 5 are considered effective to block GnRH-induced LH secretion in vitro at some reasonable concentration. All of the peptides are considered to be effective to prevent ovulation of female mammals at low dosages.

EXAMPLE 6

Peptides as indicated in TABLE 6 having the formula: Ac-$AA_1$-(4Cl)D-Phe-$AA_3$-Ser-$AA_5$-$AA_6$-Leu-ILys-Pro-D-Ala-$NH_2$ are prepared by the solid-phase procedure referred to above.

TABLE 6

| | $AA_1$ | $AA_3$ | $AA_5$ | $AA_6$ |
|---|---|---|---|---|
| 178 | β-D-2NAL | D-3PAL | Aph(bcg) | β-D-2NAL |
| 179 | " | ($6NO_2$)D-Trp | " " | (Dnp)D-His |
| 180 | " | D-Trp | " (ecg) | (4gua)D-Phe |
| 181 | dehydroPro | β-D-2NAL | Hap " | ($6NO_2$)D-Trp |
| 182 | " | β-D-1NAL | " (mcg) | D-Val |
| 183 | β-D-2NAL | (1For)D-Trp | " (tcg) | (Pr)D-Arg |
| 184 | " | " | Aph(bcg) | ($5NH_2$)D-Trp |
| 185 | dehydroPro | D-Trp | " (2mpcg) | D-Tyr |
| 186 | " | D-2PAL | " (4mpcg) | D-Nle |
| 187 | " | (1Ac)D-Trp | " (hcg) | (4F)D-Phe |
| 188 | Pro | D-3PAL | Hhp(pcg) | β-D-1NAL |
| 189 | (1For)D-Trp | " | " (chcg) | ($4NHCH_3$)D-Phe |
| 190 | β-D-2NAL | " | Aph(hcg) | (Ac)D-Orn |
| 191 | " | " | " (Ocg) | ($4NH_2$)D-Phe |
| 192 | β-D-1NAL | (6Br)D-Trp | " (tcg) | (1For)D-Trp |
| 193 | ($6CH_3$)D-Trp | D-4 PAL | " (bzcg) | D-4 PAL |

The peptides listed in Table 6 are considered effective to block GnRH-induced LH secretion in vitro at a reasonable concentration. All of the peptides are considered to be effective to prevent ovulation of female mammals at low dosages.

EXAMPLE 7

Peptides as indicated in TABLE 7 having the formula: Ac-β-D-2NAL-(4Cl)D-Phe-D-3PAL-Ser-$AA_5$-$AA_6$-Leu-$AA_8$-Pro-D-Ala-$NH_2$ are prepared by the solid-phase procedure referred to above.

TABLE 7

| | $AA_5$ | $AA_6$ | $AA_8$ |
|---|---|---|---|
| 194 | Arg | D-Aph(bcg) | Arg |
| 195 | " | " (Ocg) | " |
| 196 | Aph(bcg) | (4gua)D-Phe | ($Et_2$)Arg |
| 197 | " (2ncg) | D-Aph(2ncg) | ILys |
| 198 | " (bzcg) | " (bzcg) | Har |
| 199 | " (act) | " (act) | ILys |
| 200 | Hap(hicg) | " (hicg) | " |
| 201 | " (trcg) | D-Hap(trcg) | " |
| 202 | Hhp(bcg) | " (bcg) | " |
| 203 | " " | D-3PAL | (EtPr)Har |
| 204 | " " | D-Lys(Nic) | ($Me_2$)Arg |
| 205 | Aph(tcg) | D-Aph(tcg) | (MeBu)Arg |
| 206 | " (Sbcg) | " (Sbcg) | ILys |

The peptides listed in Table 7 are considered effective to block GnRH-induced LH secretion in vitro at a reasonable concentration. All of the peptides are considered to be effective to prevent ovulation of female mammals at low dosages.

Results of in vivo testing of selected of these antagonists are shown in the following Table A with dosages being given in micrograms per rat:

TABLE A

| Peptide No. | Dosage | Rats Ovulating | In Vitro histamine release: $ED_{50} \pm SEM(\mu g/ml)$ |
|---|---|---|---|
| 103A. | 2.5 | 0/8 | 50 ± 6.7 |
| | 1.0 | 1/17 | |
| | 0.5 | 8/18 | |
| 104A. | 5.0 | 6/11 | |
| | 2.5 | 2/3 | |
| 111A | 1.0 | 0/8 | |
| | 0.5 | 6/10 | |
| 114B. | 2.5 | 0/8 | |
| | 1.0 | 8/9 | |
| 115A. | 10 | 0/5 | |
| 116A. | 1.0 | 9/16 | |
| 117B. | 5.0 | 0/6 | |
| 127A. | 15 | 1/5 | |
| 128A. | 15 | 0/9 | |
| | 5 | 2/4 | |
| 129A. | 15 | 4/6 | 14 ± 2.1 |

The above Table A also reports the testing of various of these GnRH antagonists in an in vitro histamine release assay. All of these analogs are considered to be substantially less potent in releasing histamine than [Ac-D-2NAL$^1$, (4F)D-Phe$^2$, D-Trp$^3$, D-Arg$^6$]-GnRH for which the $ED_{50}$ was 0.17 ± 0.01 μg/ml--a very substantial advantage for these antagonists.

EXAMPLE 8

A peptide intermediate having the formula: Ac-β-D-2NAL-(4Cl)D-Phe-D-3PAL-Ser(Bzl)-Tyr(2BrZ)-D-Aph (Fmoc)-Leu-Lys (Ipr)-Pro-D-Ala-NH-[resin support] is prepared by the solid phase procedure referred to above. Following the removal of the Fmoc protection, the peptide intermediate is reacted as generally described in Example 1 using naphthyl isocyanate instead of PCI to form the napthylurea moiety with the side chain amino group of the D-Aph residue in the 6-position. Following cleavage and HPLC purification as previously described, the GnRH antagonist is tested. The peptide is considered to be effective to prevent ovulation of female mammals at low dosages.

EXAMPLE 9

A peptide intermediate having the formula: Ac-β-D-2NAL-(4Cl)D-Phe-D-3PAL-Ser(Bzl)-Tyr(2BrZ)-D-Aph(Fmoc)-Leu-Lys (Ipr)-Pro-D-Ala-NH-[resin support] is prepared by the solid phase procedure referred to above. Following the removal of the Fmoc protection, the peptide intermediate is reacted as generally described in Example 1 using naphthyl isothiocyanate instead of PCI to form the napthylthiourea moiety with the side chain amino group of the residue in the 6-position. Following cleavage and HPLC purification as previously described, the GnRH antagonist is tested. The peptide is considered to be effective to prevent ovulation of female mammals at low dosages.

EXAMPLE 10

A peptide intermediate having the formula: Ac-β-D-2NAL-(4Cl)D-Phe-D-3PAL-Ser(Bzl)-Tyr(2BrZ)-D-Aph (Fmoc)-Leu-Lys(Ipr)-Pro-D-Ala-NH-[resin support] is prepared by the solid phase procedure referred to above. Following the removal of the Fmoc protection, the peptide intermediate is first reacted using 2-bromoethyl,2'(Boc-amino)ethyl ether dissolved in DMF for 1 hour or until the ninhydrin test is negative to link the carbon atom to the side-chain amino group by the removal of the halogen to form: Q-NH-(CH2)2-O-$(CH_2)_2$-NH(Boc). This compound is then reacted as generally described in Example 1 using PCl to form the cyanoguanidino moiety with the side chain secondary amino group of the residue in the 6-position. Next the Boc protection group is removed, and the primary amino group reacts with the -OPh group to give the compound:

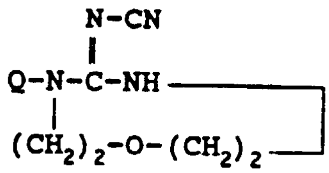

Following cleavage and HPLC purification as previously described, the GnRH antagonist is tested. The peptide is considered to be effective to prevent ovulation of female mammals at low dosages.

Following purification of the peptides, various of them are further characterized by dissolving in 0.1% TFA (10µg/10µL) and subjection to high performance liquid chromatography on $C_{18}$ silica (Vydac 0.45 x 25 cm) using a flow rate of 2.0 ml/min under isocratic conditions using a solution of 0.1% TFA in water plus the volume % (v/v) of acetonitrile indicated. The following Table B shows when the specific peptides elute from the $C_{18}$ silica having a particle size of about 5µ and a pore size of 300Å when subjected to the isocratic flow described hereinbefore, with the time given in minutes. The optical rotation measurements are taken at c=1, 50% acetic acid, at room temperature.

TABLE B

| Peptide No. | %$CH_3CN$ | Time of Elution | $[\alpha]_D$ |
|---|---|---|---|
| 103A | 30.6 | 4.48 | -33° |
| 104A | 43.2 | 4.02 | -23° |
| 111A | 38.4 | 3.75 | |
| 113B | 42.0 | 6.02 | |
| 114B | 38.4 | 4.00 | |
| 115A | 37.7 | 4.44 | |
| 116A | 31.2 | 4.01 | |
| 117B | 31.8 | 4.76 | |
| 127A | 33.6 | 4.63 | -30° |
| 128A | 39.6 | 4.19 | -30.3° |
| 129A | 40.2 | 4.20 | -42.0° |

The peptides of the invention are often administered in the form of pharmaceutically acceptable, nontoxic salts, such as acid addition salts, or of metal complexes, e.g., with zinc, barium, calcium, magnesium, aluminum or the like (which are considered as addition salts for purposes of this application), or of combinations of the two. Illustrative of such acid addition salts are hydrochloride, hydrobromide, sulphate, phosphate, nitrate, oxalate, fumarate, gluconate, tannate, maleate, acetate, citrate, benzoate, succinate, alginate, malate, ascorbate, tartrate and the like. The acetate salt is preferred. For example, an aqueous solution of the peptide can be repeatedly treated with 1N acetic acid and then lyophilized to yield the acetic acid salt thereof. If the active ingredient is to be administered in tablet form, the tablet may contain a pharmaceutically-acceptable diluent which includes a binder, such as tragacanth, corn starch or gelatin; a disintegrating agent, such as alginic acid; and a lubricant, such as magnesium stearate. If administration in liquid form is desired, sweetening and/or flavoring may be used as part of the pharmaceutically-acceptable diluent, and intravenous administration in isotonic saline, phosphate buffer solutions or the like may be effected.

The pharmaceutical compositions will usually contain the peptide in conjunctic with a conventional, pharmaceutically-acceptable carrier. Usually, the dosage will be from about 10 micrograms to about 2.5 milligrams of the peptide per kilogram of the body weight of the host when given intravenously; although oral dosages will be higher, it is anticipated that the nature of these compounds will permit effective oral administration. Overall, treatment of subjects with these peptides is generally carried out in the same manner as the clinical treatment using other antagonists of GnRH using a suitable carrier in which the peptide is soluble. Because of the favorable solubility, administration in saline is feasible.

It may also be desirable to deliver the GnRH analog over prolonged periods of time, for example, for periods of one week to one year from a single administration, and slow release, depot or implant dosage forms may be utilized.

These peptides can be administered to mammals intravenously, subcutaneously, intramuscularly, orally, percutaneously, e.g. intranasally or intravaginally to achieve fertility inhibition and/or control and also in applications calling for reversible suppression of gonadal activity, such as for the management of precocious puberty or during radiation- or chemotherapy. They are also useful for treatment of steroid-dependent tumors. Effective dosages will vary with the form of administration and the particular species of mammal being treated. An example of one typical dosage form is a bacteriostatic water solution containing the peptide which solution is administered parenterally to provide a dose in the range of about 0.1 to 2.5 mg/kg of body weight per day. Oral administration of the peptide may be given in either solid form or liquid form.

Although the invention has been described with regard to its preferred embodiments, it should be understood that changes and modifications as would be obvious to one having the ordinary skill in this art may be made without departing from the scope of the invention which is set forth in the claims which are appended hereto. For example, other substitutions known in the art which do not significantly detract from the effectiveness of the peptides may be employed in the peptides of the invention. D-2PAL and D-4PAL are considered to be equivalents of D-3PAL. Other equivalent acylating groups can be used instead of acetyl at the N-terminus. Substituted Phe, such as (4F)Phe, can be used instead of Phe in the 7-position. Both butyl Lys and diethyl Lys are considered to be equivalents of ILys; however, ILys is preferred. Har is considered the equivalent of Arg in the 8-position. Other hydrophobic amino acid residues can also be employed in the 1-position, preferably in D-isomer form, and are considered equivalents of those specified.

## Claims

**Claims for the following Contracting States : AT, BE, CH, DE, DK, FR, GB, IT, LU, NL, SE**

1. A GnRH antagonist peptide or a nontoxic salt thereof, said peptide having the formula: Ac-$AA_1$-(A)D-Phe-$AA_3$-Ser-$AA_5$-$AA_6$-$AA_7$-$AA_8$-Pro-$AA_{10}$ wherein $AA_1$ is β-D-NAL, (A)D-Phe or (B)D-Trp; A is 4Cl,4F, $4NO_2$, $4CH_3$, $4OCH_3$, $C^\alpha$ Me/4Cl, $2,4Cl_2$, 4Br or H; B is H, $6NO_2$, $6NH_2$, $6OCH_3$, 6F, 6Cl, 6Br, $6CH_3$, $N^{in}$For or $N^{in}$Ac; $AA_3$ is D-PAL, U*, β-D-NAL or (B)D-Trp; $AA_5$ is U*, Lys(cpd) or Tyr; $AA_6$ is U*, β-D-NAL, $4NH_2$D-Phe, (B)D-Trp, D-Lys(cpd), D-PAL or D-Arg, $AA_7$ is Leu, NML, Nle or Phe; $AA_8$ is ILys or Arg; $AA_{10}$ is D-Ala-$NH_2$, Gly-$NH_2$, $NHNHCONH_2$ or NH(R); R is lower alkyl; and U* is either

(a)

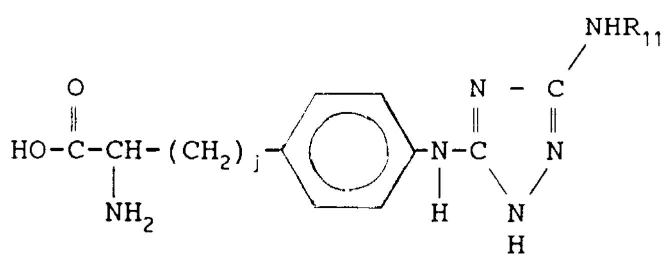

where j is 1 or 2 and $R_{11}$ is H or an acyl radical having 1 to 6 carbon atoms; or

(b)

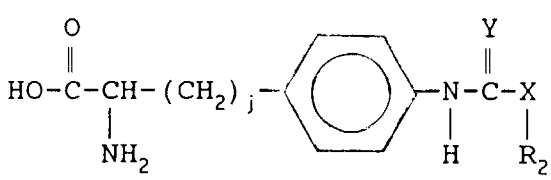

where j is 1,2 or 3; X is NH or O; Y is N-CN or N-$CONH_9$
where $R_9$ is H or lower alkyl; $R_2$ is lower alkyl, cyclohexyl, phenyl, pyridyl, methyl pyridyl or histaminyl;

provided, however, that at least one of $AA_3$, $AA_5$ and $AA_6$ is U*, with $AA_3$ and $AA_6$ always being a D-isomer.

**2.** A GnRH antagonist peptide according to Claim 1 wherein j is l, X is NH, Y is N-CN or N-$CONH_2$ and $R_{11}$ is H or acetyl.

**3.** A peptide according to Claim 1 wherein U* is (a), j is 1, and $R_{11}$ is H.

**4.** A peptide in accordance with Claim 3 wherein $AA_6$ is U*.

**5.** A peptide in accordance with Claim 3 wherein $AA_5$ is U*.

**6.** A peptide in accordance with Claim 3 wherein both $AA_5$ and $AA_6$ are U*.

**7.** A peptide in accordance with Claim 3 wherein $AA_3$ is U*.

**8.** A peptide in accordance with Claim 3 wherein $AA_1$ is β-D-2NAL, (A) is 4Cl or 4F and $AA_3$ is D-3PAL.

**9.** A peptide in accordance with Claim 8 wherein $AA_6$ is D-Trp, D-PAL, β-D-NAL, (imBzl)D-His or ($6NO_2$)D-Trp and $AA_5$ is U*.

**10.** A GnRH antagonist peptide according to Claim 1 having the formula:
Ac-β-D-2NAL-(4Cl)D-Phe-D-3PAL-Ser-Aph(3-amino 1,2,4 triazole)-D-Aph(3-amino 1,2,4 triazole)-Leu-Lys(isopropyl)-Pro-D-Ala-$NH_2$.

**11.** A peptide or a nontoxic salt thereof, said peptide having the formula:
G-$AA_1$-(A)D-Phe-$AA_3$-Ser-$AA_5$-$AA_6$-$AA_7$-$AA_8$-Pro-$AA_{10}$ wherein G is hydrogen or an acyl group having 7 or less carbon atoms; $AA_1$ is dehydroPro, D-pGlu, (A)D-Phe, (B)D-Trp, Pro, or β-D-NAL; A is H, Cl, F, $NO_2$, $CH_3$, $OCH_3$, $C^{\alpha}Me$/4Cl, $Cl_2$ or Br; B is H, $NO_2$, $NH_2$, $OCH_3$, F, Cl, Br, $CH_3$, $N^{in}$For or $N^{in}$Ac; $AA_3$ is U*, D-PAL, β-D-NAL or (B)D-Trp; $AA_5$ is U*, Lys(cpd), Orn(cpd), Dbu(cpd), Dpr(cpd), Tyr, (C)Arg, (A)Phe, (3I)Tyr or His; C is H or lower alkyl; $AA_6$ is U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala, or D-Ser(OtBu); A' is A, $NH_2$, $NHCH_3$ or gua; D is G, cpd or an aryl group; E is H, imBzl or dinitrophenol; $AA_7$ is Nle, Leu, NML, (A)Phe, Met, Nva, Tyr, (B)Trp or PAL; $AA_8$ is ILys, (C')Arg or (C')Har; C' is H or di-lower alkyl; $AA_{10}$ is D-Ala-$NH_2$, Gly-$NH_2$, $NHNHCONH_2$ or NH(R); R is lower alkyl; and U* is

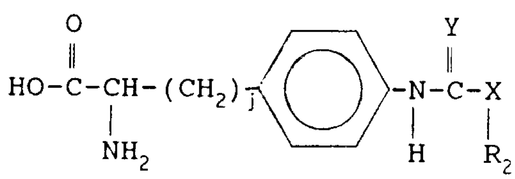

where j = 1,2 or 3; Y is N-CN, N-$CONHR_9$, O, S or CH-$NO_2$
where $R_9$ is H, Ac, alkyl, naphthyl, pyridyl, pryrimidyl, pyrazinyl, indolyl, quinolinyl or imidazolyl, which alkyl and cyclic groups are unsubstituted or substituted;
X is NH, O, $N_3$, $M_1(CH_q)_pM_2$ or $M_1$-$(CH_2)_{p'}$-$M_2(CH_2)_{p''}$-$M_3$, where $M_1$ is $NR_{10}$, N, O or $CHR_3$ wherein $R_3$ is methyl, ethyl, propyl, phenyl, pyridinyl, pyrimidinyl or purinyl; q is 1 or 2;
p, p' and p" are integers between O and 6; $R_{10}$ is H, methyl, ethyl, propyl, phenyl or substituted phenyl; and $M_2$ and $M_3$ are $M_1$, COOH, $CONH_2$, $COOR_3$ or CN; $R_1$ is H, alkyl, $(CH_2)_{n'}X''$, alkenyl, alkynyl, aryl or a direct bond to X;
n' is 1, 2, 3 or 4; X" is $CH_2NH_2$, $CH_2OH$, $CH_2Cl$, $CH_2Br$, $CH_2F$, $CF_3$ or $CF_2CF_3$; $R_2$ is $R_1$, OH, $NH_2$, $NHR_1$, heterocycle or des$R_2$, with $R_2$ being des$R_2$ when X is $N_3$; provided, however, that $R_1$ and $R_2$ are optionally interconnected via a branched or unbranched bridge so that $R_1$-$R_2$ is either -$(CH_2)_m$- or -$(CH_2)_m$-M-$(CH_2)_{m'}$-where m and m' are integers from 1 to 6 and M is NH, O, S or $CHR_4$, with $R_4$ being lower alkyl or aryl; provided further that Y and $R_2$ are optionally interconnected; and provided still further that at least one of $AA_3$,

$AA_5$ and $AA_6$ is U*, with U* being a D-isomer whenever present as $AA_3$ or $AA_6$.

**12.** A peptide in accordance with Claim 11 wherein U* is Aph(tcg).

**13.** A peptide in accordance with Claim 11 wherein U* is Aph(bcg).

**14.** A peptide in accordance with any one of Claims 11-13 wherein $AA_8$ is Arg, G is Ac and (D) and (E) are both H.

**15.** A peptide in accordance with Claim 11 wherein U* contains a cyanoguanidine moiety and wherein j is 1.

**16.** A peptide in accordance with either Claim 11 wherein $AA_5$ is Aph(tcg), $AA_6$ is D-Aph(tcg) and $AA_8$ is ILys.

**17.** A peptide in accordance with Claim 11 wherein $AA_5$ is Lys(Nic), $AA_6$ is U* and $AA_8$ is ILys.

**18.** A peptide intermediate for making a peptide according to Claim 1, which intermediate has the formula: Ac-$AA_1$-(4Cl) D-Phe-$AA_3$-Ser($X^3$) -Aph($X^a$)-D-Aph($X^a$)-Leu-Lys(Ipr,$X^6$)-Pro-D-Ala-NH-[resin support], wherein $AA_1$ is β-D-NAL, (A)D-Phe or (B)D-Trp; B is H, $6NO_2$ or $N^{in}$For; $AA_3$ is D-PAL, β-D-NAL or (B)D-Trp, $X^3$ is a protecting group for a hydroxyl group of Ser or Thr; $X^a$ is a protecting group for a primary amino group that is base-labile, hydrazine-labile or thio-labile; and $X^6$ is Z or 2ClZ.

**19.** A peptide intermediate according to Claim 18 having the formula: Ac-β-D-2NAL-(4Cl)D-Phe-D-3PAL-Ser($X^3$)-Aph($X^a$)-D-Aph(Xa)-Leu-Lys(Ipr,Z)-Pro-D-Ala-NH-[resin support].

**20.** An unnatural L- or D-isomer amino acid having the formula:

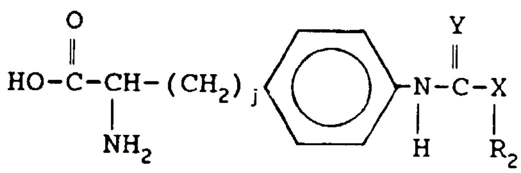

where j is 1,2 or 3; Y is N-CN, N-$CONHR_9$, O, S or CH-$NO_2$
where $R_9$ is H, Ac, alkyl, naphthyl, pyridyl, pryrimidyl, pyrazinyl, indolyl, quinolinyl or imidazolyl, which alkyl and cyclic groups are unsubstituted or substituted;
X is NH, O, $N_3$, $M_1(CH_q)_pM_2$ or $M_1$-$(CH_2)_{p'}$-$M_2(CH_2)_{p''}$-$M_3$, where $M_1$ is $NR_{10}$, N, O, or $CHR_3$ wherein $R_3$ is methyl, ethyl, propyl, phenyl, pyridinyl, pyrimidinyl or purinyl; q is 1 or 2;
p, p' and p" are integers between O and 6; $R_{10}$ is H, methyl, ethyl, propyl, phenyl or phenyl substituted by Cl, F, $NO_2$, or $NH_2$; and $M_2$ and $M_3$ are $M_1$, COOH, $CONH_2$, $COOR_3$ or CN; $R_1$ = H, alkyl ($C_1$ to $C_6$), $(CH_2)_{n'}X''$, alkenyl ($C_2$ to $C_4$), alkynyl ($C_2$ to $C_4$), aryl, or a direct bond to X; n' is 1, 2, 3 or 4; X" is $CH_2NH_2$, $CH_2OH$, $CH_2Cl$, $CH_2Br$, $CH_2F$, $CF_3$ or $CF_2CF_3$; $R_2$ is $R_1$, OH, $NH_2$, $NHR_1$, heterocycle or des$R_2$, with $R_2$ being des$R_2$ when X is $N_3$; provided, however, that $R_1$ and $R_2$ are optionally interconnected via a branched or unbranched bridge so that $R_1$-$R_2$ is either -$(CH_2)_m$- or -$(CH_2)_m$-M-$(CH_2)_m$, where m and m' are integers from 1 to 6 and M is NH, O, S or $CHR_4$, with $R_4$ being lower alkyl or aryl; provided further that Y and $R_2$ are optionally interconnected.

**21.** A method for making a peptide which includes a residue U* of an unnatural amino acid according to Claim 20, which peptide has the formula: G-$AA_1$-(A)D-Phe-$AA_3$-Ser-$AA_5$-$AA_6$-$AA_7$-$AA_8$-Pro-$AA_{10}$ wherein G is hydrogen or an acyl group having 7 or less carbon atoms; $AA_1$ is dehydroPro, D-pGlu, (A)D-Phe, (B)D-Trp, Pro, or β-D-NAL; A is H, Cl, F, $NO_2$, $CH_3$, $OCH_3$, $C^{\alpha}$Me/4Cl, $Cl_2$ or Br; B is H, $NO_2$, $NH_2$, $OCH_3$, F, Cl, Br, $CH_3$, $N^{in}$For or $N^{in}$Ac; $AA_3$ is U*, D-PAL, β-D-NAL or (B)D-Trp; $AA_5$ is U*, Lys(cpd), Orn(cpd), Dbu(cpd), Dpr(cpd), Tyr, (C)Arg, (A)Phe, (3I) Tyr or His; C is H or lower alkyl; $AA_6$ is U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala, or D-Ser(OtBu); A' is A, $NH_2$, $NHCH_3$ or gua; D is G, cpd or an aryl group; E is H, imBzl or dinitrophenol; $AA_7$ is Nle, Leu, NML, (A)Phe, Met, Nva, Tyr, (B)Trp or PAL; $AA_8$ is ILys, (C')Arg or (C')Har; C' is H or di-lower alkyl; $AA_{10}$ is D-Ala-$NH_2$, Gly-$NH_2$, $NHNHCONH_2$ or NH(R); R is lower alkyl; provided, however, that at least one of $AA_3$, $AA_5$, and $AA_6$ is U*, which method comprises

(a) forming an intermediate peptide having the formula:
$X^1$-$AA_1$-(A)D-Phe-$U_3$-Ser($X^3$)-$U_5$-$U_6$-$AA_7$($X^2$ or $X^7$)-$AA_8$($X^5$ or $X^6$)-Pro-$X^8$ wherein: $U_3$ is either U' or $AA_3$($X^2$); $U_5$ is either U' or $AA_5$ ($X^4$ or $X^5$); $U_6$ is either U' or $AA_6$($X^4$ or $X^5$ or $X^6$); U' is Aph($X^a$), Hap($X^a$), or Hhp($X^a$); $X^1$ is hydrogen or an $\alpha$-amino protecting group; $X^2$ is hydrogen or a protecting group for an indole nitrogen; $X^3$ is a protecting group for a hydroxyl group of Ser or Thr; $X^4$ is hydrogen or a protecting group for a phenolic hydroxyl group of Tyr; $X^5$ is either hydrogen or a protecting group for a guanidino or imidazole group; $X^6$ is a protecting group for a primary amino group; $X^a$ is a protecting group for a primary amino group that is base-labile, hydrazine-labile or thio-labile; $X^7$ is hydrogen or a protecting group for Met; $X^8$ is Gly-NH-[resin support], D-Ala-NH-[resin support], N(A)-[resin support], an amide either of Gly or of D-Ala or a substituted amide attached directly to Pro; provided however that at least one of $U_3$, $U_5$ and $U_6$ is Aph($X^a$), Hap($X^a$), or Hhp($X^a$), with $U_3$ and $U_6$ always being a D-isomer; (b) removing at least one $X^a$ to deprotect a side chain primary amino group of at least one amino acid residue of said intermediate peptide; (c) reacting said deprotected side chain primary amino group to build said residue into one having the formula U*; and (d) splitting off any remaining groups $X^1$ to $X^7$ and/or cleaving from any resin support included in $X^8$.

**22.** A method in accordance with Claim 21 wherein either $U_5$ is U' or $U_6$ is U' or both are U'.

**Claims for the following Contracting States : ES, GR**

**1.** A method for making a GnRH antagonist peptide or a nontoxic salt thereof, said peptide having the formula: Ac-$AA_1$-(A) D-Phe-$AA_3$-Ser-$AA_5$-$AA_6$-$AA_7$-$AA_8$-pro-$AA_{10}$ wherein $AA_1$ is β-D-NAL, (A)D-Phe or (B)D-Trp; A is 4Cl,4F, $4NO_2$, $4CH_3$, $4OCH_3$, $C^{\alpha}Me$/4Cl, 2,$4Cl_2$, 4Br or H; B is H, $6NO_2$, $6NH_2$, $6OCH_3$, 6F, 6Cl, 6Br, $6CH_3$, $N^{in}$For or $N^{in}$Ac; $AA_3$ is D-PAL, U*, β-D-NAL or (B)D-Trp; $AA_5$ is U*, Lys(cpd) or Tyr; $AA_6$ is U*, β-D-NAL, $4NH_2$D-Phe, (B) D-Trp, D-Lys(cpd), D-PAL or D-Arg, $AA_7$ is Leu, NML, Nle or Phe; $AA_8$ is ILys or Arg; $AA_{10}$ is D-Ala-$NH_2$, Gly-$NH_2$, $NHNHCONH_2$ or NH(R); R is lower alkyl; and U* is either

(a)

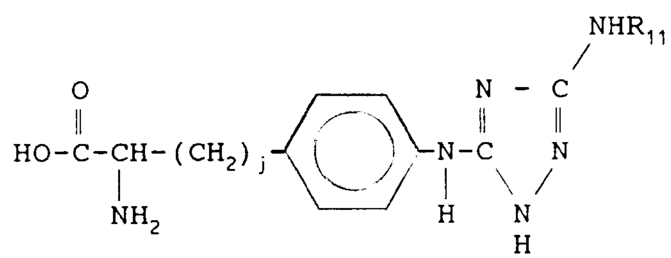

where j is 1 or 2 and $R_{11}$ is H or an acyl radical having 1 to 6 carbon atoms;
or

(b)

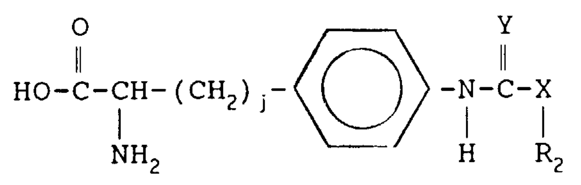

where j is 1,2 or 3; X is NH or O; Y is N-CN or N-$CONH_9$
where $R_9$ is H or lower alkyl; $R_2$ is lower alkyl, cyclohexyl, phenyl, pyridyl, methylpyridyl or histaminyl;

provided, however, that at least one of $AA_3$, $AA_5$ and $AA_6$ is U*, with $AA_3$ and $AA_6$ always being a D-isomer, which method comprises (i) forming an intermediate peptide having the formula: $X^1$-$AA_1$-(A)D-Phe-$U_3$-Ser($X^3$)-$U_5$-$U_6$-$AA_7$($X^2$ or $X^7$)-$AA_8$($X^5$ or $X^6$)-Pro-$X^8$ wherein: $U_3$ is either U' or $AA_3$($X^2$) ; $U_5$ is either U' or $AA_5$($X^4$ or $X^5$) ; $U_6$ is either U' or $AA_6$($X^4$ or $X^5$ or $X^6$); U' is either Aph($X^a$), Hap($X^a$) or Hhp($X^a$); $X^1$ is hydrogen or an α-amino protecting group; $X^2$ is hydrogen or a protecting group for an indole nitrogen; $X^3$ is a protecting group for a hydroxyl group of Ser or Thr; $X^4$ is hydrogen or a protecting group for a phenolic hydroxyl group of Tyr; $X^5$ is either hydrogen or a protecting group for a guanidino or imidazole side chain; $X^6$ is a protecting group for a primary amino side chain of which $X^a$ is a subgroup that is removable without removing other protecting groups; $X^7$ is hydrogen or a protecting group for Met; $X^8$ is Gly-NH-[resin support], D-Ala-NH-[resin support], N(A)-[resin support], an amide either of Gly or of D-Ala or a substituted amide attached directly to Pro or $NHNHCONH_2$; provided however that at least one of $U_3$, $U_5$ and $U_6$ is either Aph($X^a$), Hap($X^a$), or Hhp($X^a$); (ii) removing at least one $X^a$ to deprotect a side chain primary amino group of at least one amino acid residue of said intermediate peptide; (iii) reacting said deprotected side chain primary amino group to build said residue into one having the formula U*; (iv) splitting off any remaining groups $X^1$ to $X^7$ and/or cleaving from any resin support included in $X^8$, and (v) optionally converting said peptide to a nontoxic salt thereof.

**2.** A method according to Claim 1 wherein j is 1, X is NH, Y is N-CN or N-$CONH_2$ and $R_{11}$ is H or acetyl.

**3.** A method according to Claim 1 wherein U* is (a), j is 1 and $R_{11}$ is H.

**4.** A method in accordance with Claim 3 wherein $AA_6$ is U*.

**5.** A peptide in accordance with Claim 3 wherein $AA_5$ is U*.

**6.** A method in accordance with Claim 3 wherein both $AA_5$ and $AA_6$ are U*.

**7.** A method in accordance with Claim 3 wherein $AA_3$ is U*.

**8.** A method in accordance with Claim 3 wherein $AA_1$ is β-D-2NAL, (A) is 4Cl or 4F and $AA_3$ is D-3PAL.

**9.** A method in accordance with Claim 8 wherein $AA_6$ is D-Trp, D-PAL, β-D-NAL, (imBzl)D-His or ($6NO_2$)D-Trp and $AA_5$ is U*.

**10.** A method according to Claim 1 wherein said peptide has the formula:
Ac-β-D-2NAL-(4Cl)D-Phe-D-3PAL-Ser-Aph(3-amino 1,2,4 triazole)-D-Aph(3-amino 1,2,4 triazole)-Leu-Lys(isopropyl)-Pro-D-Ala-$NH_2$.

**11.** A method for making a peptide or a nontoxic salt thereof, said peptide having the formula: G-$AA_1$-(A)D-Phe-$AA_3$-Ser-$AA_5$-$AA_6$-$AA_7$-$AA_8$-Pro-$AA_{10}$ wherein G is hydrogen or an acyl group having 7 or less carbon atoms; $AA_1$ is dehydroPro, D-pGlu, (A)D-Phe, (B)D-Trp, Pro, or β-D-NAL; A is H, Cl, F, $NO_2$, $CH_3$, $OCH_3$, $C^{\alpha}Me$/4Cl, $Cl_2$ or Br; B is H, $NO_2$, $NH_2$, $OCH_3$, F, Cl, Br, $CH_3$, $N^{in}$For or $N^{in}$Ac; $AA_3$ is U*, D-PAL, β-D-NAL or (B)D-Trp; $AA_5$ is U*, Lys(cpd), Orn(cpd), Dbu(cpd), Dpr(cpd), Tyr, (C)Arg, (A)Phe, (3I)Tyr or His; C is H or lower alkyl; $AA_6$ is U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala, or D-Ser(OtBu); A' is A, $NH_2$, $NHCH_3$ or gua; D is G, cpd or an aryl group; E is H, imBzl or dinitrophenol; $AA_7$ is Nle, Leu, NML, (A)Phe, Met, Nva, Tyr, (B)Trp or PAL; $AA_8$ is ILys, (C')Arg or (C')Har; C' is H or di-lower alkyl; $AA_{10}$ is D-Ala-$NH_2$, Gly-$NH_2$, $NHNHCONH_2$ or NH(R); R is lower alkyl; and U* is

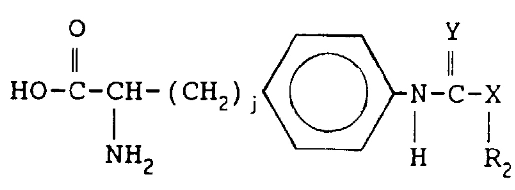

where j = 1,2 or 3; Y is N-CN, N-$CONHR_9$, O, S or CH-$NO_2$
where $R_9$ is H, Ac, alkyl, naphthyl, pyridyl, pryrimidyl, pyrazinyl, indolyl, quinolinyl or imidazolyl, which alkyl and cyclic groups are unsubstituted or substituted;

X is NH, O, $N_3$, $M_1(CH_q)_pM_2$ or $M_1$-$(CH_2)_{p'}$-$M_2(CH_2)_{p''}$-$M_3$, where $M_1$ is $NR_{10}$, N, O or $CHR_3$ wherein $R_3$ is methyl, ethyl, propyl, phenyl, pyridinyl, pyrimidinyl or purinyl; q is 1 or 2;
p, p' and p" are integers between O and 6; $R_{10}$ is H, methyl, ethyl, propyl, phenyl or substituted phenyl; and $M_2$ and $M_3$ are $M_1$, COOH, $CONH_2$, $COOR_3$ or CN; $R_1$ is H, alkyl, $(CH_2)_{n'}X''$, alkenyl, alkynyl, aryl or a direct bond to X;
n' is 1, 2, 3 or 4; X" is $CH_2NH_2$, $CH_2OH$, $CH_2Cl$, $CH_2Br$, $CH_2F$, $CF_3$ or $CF_2CF_3$; $R_2$ is $R_1$, OH, $NH_2$, $NHR_1$, heterocycle or des$R_2$, with $R_2$ being des$R_2$ when X is $N_3$; provided, however, that $R_1$ and $R_2$ are optionally interconnected via a branched or unbranched bridge so that $R_1$-$R_2$ is either -$(CH_2)_m$- or -$(CH_2)_m$-M-$(CH_2)_{m'}$- where m and m' are integers from 1 to 6 and M is NH, O, S or $CHR_4$, with $R_4$ being lower alkyl or aryl; provided further that Y and $R_2$ are optionally interconnected; and provided still further that at least one of $AA_3$, $AA_5$ and $AA_6$ is U*, with U* being a D-isomer whenever present as $AA_3$ or $AA_6$;

which method comprises (i) forming an intermediate peptide having the formula: $X^1$-$AA_1$-(A)D-Phe-$U_3$-Ser($X^3$)-$U_5$- $U_6$-$AA_7$($X^2$ or $X^7$)-$AA_8$($X^5$ or $X^6$)-Pro-$X^8$ wherein: $U_3$ is either U' or $AA_3$($X^2$) ; $U_5$ is either U' or $AA_5$($X^4$ or $X^5$); $U_6$ is either U' or $AA_6$($X^4$ or $X^5$ or $X^6$); U' is either Aph($X^a$), Hap($X^a$) or Hhp($X^a$); $X^1$ is hydrogen or an $\alpha$-amino protecting group; $X^2$ is hydrogen or a protecting group for an indole nitrogen; $X^3$ is a protecting group for a hydroxyl group of Ser or Thr; $X^4$ is hydrogen or a protecting group for a phenolic hydroxyl group of Tyr; $X^5$ is either hydrogen or a protecting group for a guanidino or imidazole side chain; $X^6$ is a protecting group for a primary amino side chain of which $X^a$ is a subgroup that is removable without removing other protecting groups; $X^7$ is hydrogen or a protecting group for Met; $X^8$ is Gly-NH-[resin support], D-Ala-NH-[resin support], N(A)-[resin support], an amide either of Gly or of D-Ala or a substituted amide attached directly to Pro or $NHNHCONH_2$; provided however that at least one of $U_3$, $U_5$ and $U_6$ is either Aph($X^a$), Hap($X^a$), or Hhp($X^a$); (ii) removing at least one $X^a$ to deprotect a side chain primary amino group of at least one amino acid residue of said intermediate peptide; (iii) reacting said deprotected side chain primary amino group to build said residue into one having the formula U*; (iv) splitting off any remaining groups $X^1$ to $X^7$ and/or cleaving from any resin support included in $X^8$, and (v) optionally converting said peptide to a nontoxic salt thereof.

**12.** A method in accordance with Claim 11 wherein U* is Aph(tcg).

**13.** A method in accordance with Claim 11 wherein U* is Aph(bcg).

**14.** A method in accordance with any one of Claims 11-13 wherein $AA_8$ is Arg, G is Ac and (D) and (E) are both H.

**15.** A method in accordance with Claim 11 wherein U* contains a cyanoguanidine moiety and wherein j is 1.

**16.** A method in accordance with Claim 11 wherein $AA_5$ is Aph(tcg), $AA_6$ is D-Aph(tcg) and $AA_8$ is ILys.

**17.** A method in accordance with Claim 11 wherein $AA_5$ is Lys(Nic), $AA_6$ is U* and $AA_8$ is ILys.

**18.** A method for making a peptide according to Claim 1, wherein said intermediate has the formula: Ac-$AA_1$-(4Cl) D-Phe-$AA_3$-Ser($X^3$) -Aph($X^a$) -D-Aph ($X^a$)-Leu-Lys(Ipr,$X^6$)-Pro-D-Ala-NH-[resin support], wherein $AA_1$ is $\beta$-D-NAL, (A)D-Phe or (B)D-Trp; B is H, $6NO_2$ or $N^{in}$For; $AA_3$ is D-PAL, $\beta$-D-NAL or (B)D-Trp, $X^3$ is a protecting group for a hydroxyl group of Ser or Thr; $X^a$ is a protecting group for a primary amino group that is base-labile, hydrazine-labile or thio-labile; and $X^6$ is Z or 2ClZ.

**19.** A method according to Claim 18 wherein said intermediate has the formula: Ac-$\beta$-D-2NAL-(4Cl)D-Phe-D-3PAL-Ser($X^3$)Aph($X^a$)-D-APh($X^a$)-Leu-Lys(Ipr,Z)-Pro-D-Ala-NH-[resin support].

**20.** An L- or D-isomer amino acid for use in a method for making a peptide, which amino acid has the formula:

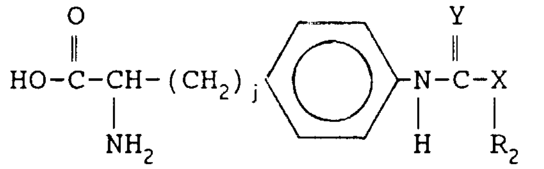

where j is 1,2 or 3; Y is N-CN, N-CONHR$_9$, O, S or CH-NO$_2$
where R$_9$ is H, Ac, alkyl, naphthyl, pyridyl, pryrimidyl, pyrazinyl, indolyl, quinolinyl or imidazolyl, which alkyl and cyclic groups are unsubstituted or substituted;
X is NH, O, N$_3$, $M_1(CH_q)_pM_2$ or $M_1$-$(CH_2)_{p'}$-$M_2(CH_2)_{p''}$-$M_3$, where $M_1$ is $NR_{10}$, N, O, or $CHR_3$ wherein $R_3$ is methyl, ethyl, propyl, phenyl, pyridinyl, pyrimidinyl or purinyl; q is 1 or 2;
p, p' and p" are integers between O and 6; $R_{10}$ is H, methyl, ethyl, propyl, phenyl or phenyl substituted by Cl, F, $NO_2$, or $NH_2$; and $M_2$ and $M_3$ are $M_1$, COOH, $CONH_2$, $COOR_3$ or CN; $R_1$ = H, alkyl ($C_1$ to $C_6$), $(CH_2)_{n'}X''$, alkenyl ($C_2$ to $C_4$), alkynyl ($C_2$ to $C_4$), aryl, or a direct bond to X; n' is 1, 2, 3 or 4; X" is $CH_2NH_2$, $CH_2OH$, $CH_2Cl$, $CH_2Br$, $CH_2F$, $CF_3$ or $CF_2CF_3$; $R_2$ is $R_1$, OH, $NH_2$, $NHR_1$, heterocycle or des$R_2$, with $R_2$ being des$R_2$ when X is $N_3$; provided, however, that $R_1$ and $R_2$ are optionally interconnected via a branched or unbranched bridge so that $R_1$-$R_2$ is either $-(CH_2)_m-$ or $-(CH_2)_m-M-(CH_2)_{m'}$ where m and m' are integers from 1 to 6 and M is NH, O, S or $CHR_4$, with $R_4$ being lower alkyl or aryl; provided further that Y and $R_2$ are optionally interconnected.

## Patentansprüche

**Patentansprüche für folgende Vertragsstaaten : AT, BE, CH, DE, DK, FR, GB, IT, LU, NL, SE**

**1.** GnRH-Antagonist-Peptid oder nichttoxisches Salz davon, welches Peptid die folgende Formel hat:
Ac-AA$_1$-(A)D-Phe-AA$_3$-Ser-AA$_5$-AA$_6$-AA$_7$-AA$_8$-Pro-AA$_{10}$, worin AA$_1$ β-D-NAL, (A)D-Phe oder (B)D-Trp ist; A 4Cl, 4F, 4NO$_2$, 4CH$_3$, 4OCH$_3$, C$^\alpha$Me/4Cl, 2,4Cl$_2$, 4Br oder H ist; B H, 6NO$_2$, 6NH$_2$, 6OCH$_3$, 6F, 6Cl, 6Br, 6CH$_3$, N$^{in}$For oder N$^{in}$Ac ist; AA$_3$ D-PAL, U*, β-D-NAL oder (B)D-Trp ist; AA$_5$ U*, Lys(cpd) oder Tyr ist; AA$_6$ U*, β-D-NAL, 4NH$_2$D-Phe, (B)D-Trp, D-Lys(cpd), D-PAL oder D-Arg ist; AA$_7$ Leu, NML, Nle oder Phe ist; AA$_8$ ILys oder Arg ist; AA$_{10}$ D-Ala-NH$_2$, Gly-NH$_2$, NHNHCONH$_2$ oder NH(R) ist; R Niedrigalkyl ist; und U* entweder

(a)

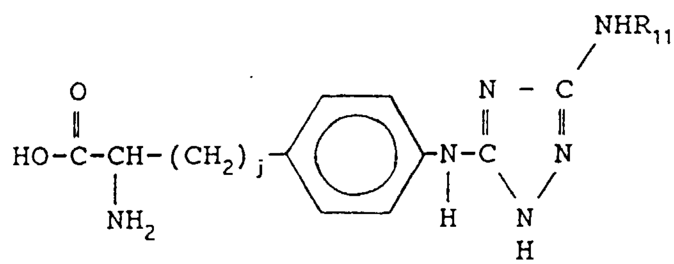

worin j 1 oder 2 und R$_{11}$ H oder ein Acylrest mit 1 bis 6 Kohlenstoffatomen ist;
oder

(b)

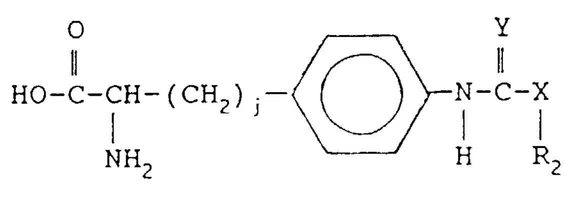

worin j 1, 2 oder 3 ist; X NH oder O ist; Y N-CN oder N-CONHR$_9$ ist, worin R$_9$ H oder Niedrigalkyl ist; R$_2$ Niedrigalkyl, Cyclohexyl, Phenyl, Pyridyl, Methylpyridyl oder Histaminyl ist;
jedoch mit der Maßgabe, daß zumindest eines von AA$_3$, AA$_5$ und AA$_6$ U* ist, wobei AA$_3$ und AA$_6$ immer ein D-Isomer sind.

**2.** GnRH-Antagonist-Peptid nach Anspruch 1, worin j 1 ist, X NH ist, Y N-CN oder N-$CONH_2$ ist und $R_{11}$ H oder Acetyl ist.

**3.** Peptid nach Anspruch 1, worin U* (a) ist, j 1 ist und $R_{11}$ H ist.

**4.** Peptid nach Anspruch 3, worin $AA_6$ U* ist.

**5.** Peptid nach Anspruch 3, worin $AA_5$ U* ist.

**6.** Peptid nach Anspruch 3, worin sowohl $AA_5$ als auch $AA_6$ U* sind.

**7.** Peptid nach Anspruch 3, worin $AA_3$ U* ist.

**8.** Peptid nach Anspruch 3, worin $AA_1$ β-D-2NAL ist, (A) 4Cl oder 4F ist und $AA_3$ D-3PAL ist.

**9.** Peptid nach Anspruch 8, worin $AA_6$ D-Trp, D-PAL, β-D-NAL, (imBzl)D-His oder ($6NO_2$)D-Trp ist und $AA_5$ U* ist.

**10.** GnRH-Antagonist-Peptid nach Anspruch 1 der Formel:
Ac-β-D-2NAL-(4Cl)D-Phe-D-3PAL-Ser-Aph(3-amino-1,2,4-triazol)-D-Aph(3-amino-1,2,4-triazol)-Leu-Lys(isopropyl)-Pro-D-Ala-$NH_2$.

**11.** Peptid oder nichttoxisches Salz desselben, welches Peptid die folgende Formel hat:
G-$AA_1$-(A)D-Phe-$AA_3$-Ser-$AA_5$-$AA_6$-$AA_7$-$AA_8$-Pro-$AA_{10}$, worin G Wasserstoff oder eine Acylgruppe mit 7 oder weniger Kohlenstoffatomen ist; $AA_1$ dehydroPro, D-pGlu, (A)D-Phe, (B)D-Trp, Pro oder β-D-NAL ist; A H, Cl, F, $NO_2$, $CH_3$, $OCH_3$, $C^{\alpha}Me$/4Cl, $Cl_2$ oder Br ist; B H, $NO_2$, $NH_2$, $OCH_3$, F, Cl, Br, $CH_3$, $N^{in}$For oder $N^{in}$Ac ist; $AA_3$ U*, D-PAL, β-D-NAL oder (B)D-Trp ist; $AA_5$ U*, Lys(cpd), Orn(cpd), Dbu(cpd), Dpr(cpd), Tyr, (C)Arg, (A)Phe, (3I)Tyr oder His ist; C H oder Niedrigalkyl ist; $AA_6$ U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala oder D-Ser(OtBu) ist; A' A, $NH_2$, $NHCH_3$ oder gua ist; D G, cpd oder eine Arylgruppe ist; E H, imBzl oder Dinitrophenol ist; $AA_7$ Nle, Leu, NML, (A)Phe, Met, Nva, Tyr, (B)Trp oder PAL ist; $AA_8$ ILys, (C')Arg oder (C')Har ist; C' H oder Di-Niedrigalkyl ist; AA10 D-Ala-NH2, Gly-$NH_2$, $NHNHCONH_2$ oder NH(R) ist; R Niedrigalkyl ist; und U*

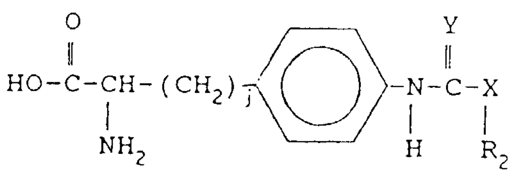

ist, worin j 1, 2 oder 3 ist; Y N-CN, N-$CONHR_9$, O, S oder CH-$NO_2$ ist, worin $R_9$ H, Ac, Alkyl, Naphthyl, Pyridyl, Pyrimidyl, Pyrazinyl, Indolyl, Chinolinyl oder Imidazolyl ist, welche Alkyl- und zyklischen Gruppen nichtsubstituiert oder substituiert sind;
X NH, O, $N_3$, $M_1(CH_q)_pM_2$ oder $M_1(CH_2)_{p'}$-$M_2(CH_2)_{p''}$-$M_3$ ist, worin $M_1$ $NR_{10}$, N, O oder $CHR_3$ ist, worin $R_3$ Methyl, Ethyl, Propyl, Phenyl, Pyridinyl, Pyrimidinyl oder Purinyl ist; q 1 oder 2 ist; p, p' und p" ganze Zahlen zwischen 0 und 6 sind; $R_{10}$ H, Methyl, Ethyl, Propyl, Phenyl oder substituiertes Phenyl ist; und $M_2$ und $M_3$ $M_1$, COOH, $CONH_2$, $COOR_3$ oder CN sind; $R_1$ H, Alkyl, $(CH_2)_{n'}X''$, Alkenyl, Alkinyl, Aryl oder eine Direktbindung an X ist; n' 1, 2, 3 oder 4 ist; X" $CH_2NH_2$, $CH_2OH$, $CH_2Cl$, $CH_2Br$, $CH_2F$, $CF_3$, oder $CF_2CF_3$ ist; $R_2$ $R_1$, OH, $NH_2$, $NHR_1$, Heterozyklus oder des$R_2$ ist, wobei $R_2$ des$R_2$ ist, wenn X $N_3$ ist; jedoch mit der Maßgabe, daß $R_1$ und $R_2$ gegebenenfalls über eine verzweigte oder unverzweigte Brücke miteinander verbunden sind, so daß $R_1$-$R_2$ entweder -$(CH_2)_m$-oder -$(CH_2)_m$-M-$(CH_2)_{m'}$- ist, wobei m und m' ganze Zahlen von 1 bis 6 sind und M NH, O, S oder $CHR_4$ ist, wobei $R_4$ Niedrigalkyl oder Aryl ist; mit der weiteren Maßgabe, daß Y und $R_2$ gegebenenfalls miteinander verbunden sind; und mit der noch weiteren Maßgabe, daß zumindest eines von $AA_3$, $AA_5$ und $AA_6$ U* ist, wobei U* immer ein D-Isomer ist, wenn es als $AA_3$ oder $AA_6$ anwesend ist.

**12.** Peptid nach Anspruch 11, worin U* Aph(tcg) ist.

**13.** Peptid nach Anspruch 11, worin U* Aph(bcg) ist.

**14.** Peptid nach einem der Ansprüche 11 bis 13, worin $AA_8$ Arg ist, G Ac ist und (D) und (E) beide H sind.

**15.** Peptid nach Anspruch 11, worin U* einen Cyanoguanidinteil enthält und j 1 ist.

**16.** Peptid nach Anspruch 11, worin $AA_5$ Aph(tcg) ist, $AA_6$ D-Aph(tcg) ist und $AA_8$ ILys ist.

**17.** Peptid nach Anspruch 11, worin $AA_5$ Lys(Nic) ist, $AA_6$ U* ist und $AA_8$ ILys ist.

**18.** Peptid-Zwischenprodukt zur Herstellung eines Peptids nach Anspruch 1, welches Zwischenprodukt die folgende Formel hat: Ac-$AA_1$-(4Cl)D-Phe-$AA_3$-Ser($X^3$)-Aph($X^a$)-D-Aph($X^a$) -Leu-Lys(Ipr,$X^6$)-Pro-D-Ala-NH-[Harzträger], worin $AA_1$ β-D-NAL, (A)D-Phe oder (B)D-Trp ist; B H, $6NO_2$ oder $N^{in}$For ist; $AA_3$ D-PAL, β-D-NAL oder (B)D-Trp ist; $X^3$ eine Schutzgruppe für eine Hydroxylgruppe von Ser oder Thr ist; $X^a$ eine Schutzgruppe für eine primäre Aminogruppe ist, die basenlabil, hydrazinlabil oder thiolabil ist; und $X^6$ Z oder 2ClZ ist.

**19.** Peptid-Zwischenprodukt nach Anspruch 18 der folgenden Formel: Ac-β-D-2NAL-(4Cl)D-Phe-D-3PAL-Ser($X^3$)-Aph ($X^a$)-D-Aph($X^a$)-Leu-Lys(Ipr,Z)-Pro-D-Ala-NH-[Harzträger].

**20.** Nichtnatürliche L- oder D-isomere Aminosäure der folgenden Formel:

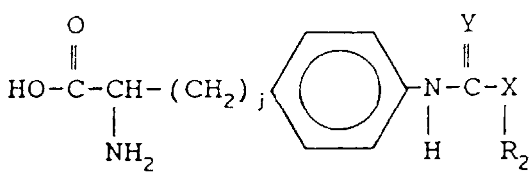

worin j 1,2 oder 3 ist; Y N-CN, N-$CONHR_9$, O, S oder CH-$NO_2$ ist, worin $R_9$ H, Ac, Alkyl, Naphthyl, Pyridyl, Pyrimidyl, Pyrazinyl, Indolyl, Chinolinyl oder Imidazolyl ist, welche Alkyl- und zyklischen Gruppen nichtsubstitiert oder substitutiert sind; X NH, O, $N_3$, $M_1(CH_q)_pM_2$ oder $M_1$-$(CH_2)_{p'}$-$M_2(CH_2)_{p''}$-$M_3$ ist, worin $M_1$ $NR_{10}$, N, O oder $CHR_3$ ist, worin $R_3$ Methyl, Ethyl, Propyl, Phenyl, Pyridinyl, Pyrimidinyl oder Purinyl ist; q 1 oder 2 ist; p, p' und p" ganze Zahlen zwischen 0 und 6 sind; $R_{10}$ H, Methyl, Ethyl, Propyl, Phenyl oder durch Cl, F, $NO_2$, oder $NH_2$ substituiertes Phenyl ist; und $M_2$ und $M_3$ $M_1$, COOH, $CONH_2$, $COOR_3$ oder CN sind; $R_1$ H, Alkyl ($C_1$ bis $C_6$), $(CH_2)_{n'}$X", Alkenyl ($C_2$ bis $C_4$), Alkinyl ($C_2$ bis $C_4$), Aryl oder eine Direktbindung an X ist; n' 1, 2, 3 oder 4 ist; X" $CH_2NH_2$, $CH_2OH$, $CH_2Cl$, $CH_2Br$, $CH_2F$, $CF_3$ oder $CF_2CF_3$ ist; $R_2$ $R_1$, OH, $NH_2$, $NHR_1$, Heterozyklus oder des$R_2$ ist, wobei $R_2$ des$R_2$ ist, wenn X $N_3$ ist; jedoch mit der Maßgabe, daß $R_1$ und $R_2$ gegebenenfalls über eine verzweigte oder unverzweigte Brücke miteinander verbunden sind, so daß $R_1$-$R_2$ entweder -$(CH_2)_m$- oder -$(CH_2)_m$-M-$(CH_2)_{m'}$- ist, wobei m und m' ganze Zahlen von 1 bis 6 sind und M NH, O, S oder $CHR_4$ ist, wobei $R_4$ Niedrigalkyl oder Aryl ist; mit der weiteren Maßgabe, daß Y und $R_2$ gegebenenfalls miteinander verbunden sind.

**21.** Verfahren zur Herstellung eines Peptids, welches einen Rest U* einer nichtnatürlichen Aminosäure gemäß Anspruch 20 enthält, welches Peptid die folgende Formel hat: G-$AA_1$-(A)D-Phe-$AA_3$-Ser-$AA_5$-$AA_6$-$AA_7$-$AA_8$-Pro-$AA_{10}$, worin G Wasserstoff oder eine Acylgruppe mit 7 oder weniger Kohlenstoffatomen ist; $AA_1$ dehydroPro, D-pGlu, (A)D-Phe, (B)D-Trp, Pro oder β-D-NAL ist; A H, Cl, F, $NO_2$, $CH_3$, $OCH_3$, $C^{\alpha}$Me/4Cl, $Cl_2$ oder Br ist; B H, $NO_2$, $NH_2$, $OCH_3$, F, Cl, Br, $CH_3$, $N^{in}$For oder $N^{in}$Ac ist; $AA_3$ U*, D-PAL, β-D-NAL oder (B)D-Trp ist; $AA_5$ U*, Lys (cpd), Orn(cpd), Dbu(cpd), Dpr(cpd), Tyr, (C)Arg, (A)Phe, (3I)Tyr oder His ist; C H oder Niedrigalkyl ist; $AA_6$ U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala oder D-Ser(OtBu) ist; A' A, $NH_2$, $NHCH_3$ oder gua ist; D G, cpd oder eine Arylgruppe ist; E H, imBzl oder Dinitrophenol ist; $AA_7$ Nle, Leu, NML, (A)Phe, Met, Nva, Tyr, (B)Trp oder PAL ist; $AA_8$ ILys, (C') Arg oder (C')Har ist; C' H oder Di-Niedrigalkyl ist; $AA_{10}$ D-Ala-$NH_2$, Gly-$NH_2$, $NHNHCONH_2$ oder NH(R) ist; R Niedrigalkyl ist; jedoch mit der Maßgabe, daß zumindest eines von $AA_3$, $AA_5$ und $AA_6$ U* ist, welches Verfahren umfaßt:

(a) Bilden eines intermediären Peptids der Formel:
$X^1$-$AA_1$-(A)D-Phe-$U_3$-Ser($X^3$)-$U_5$-$U_6$-$AA_7$-($X^2$ oder $X^7$)-$AA_8$-($X^5$ oder $X^6$)-Pro-$X^8$, worin $U_3$ entweder U' oder $AA_3$($X^2$) ist; $U_5$ entweder U' oder $AA_5$($X^4$ oder $X^5$) ist; $U_6$ entweder U' oder $AA_6$($X^4$ oder $X^5$ oder $X^6$) ist; U' Aph ($X^a$), Hap($X^a$) oder Hhp($X^a$) ist; $X^1$ Wasserstoff oder eine α-Amino-Schutzgruppe ist; $X^2$ Wasserstoff oder eine

Schutzgruppe für einen Indolstickstoff ist; $X^3$ eine Schutzgruppe für eine Hydroxylgruppe von Ser oder Thr ist; $X^4$ Wasserstoff oder eine Schutzgruppe für eine phenolische Hydroxylgruppe von Tyr ist; $X^5$ entweder Wasserstoff oder eine Schutzgruppe für eine Guanidino- oder Imidazolgruppe ist; $X^6$ eine Schutzgruppe für eine primäre Aminogruppe ist, $X^a$ eine Schutzgruppe für eine basenlabile, hydrazinlabile oder thiolabile primäre Aminogruppe ist; $X^7$ Wasserstoff oder eine Schutzgruppe für Met ist; $X^8$ Gly-NH-[Harzträger], D-Ala-NH-[Harzträger], N(A)-[Harzträger], ein Amid entweder von Gly oder von D-Ala oder ein direkt an Pro angefügtes substituiertes Amid ist; jedoch mit der Maßgabe, daß zumindest eines von $U_3$, $U_5$ und $U_6$ Aph($X^a$), Hap($X^a$) oder Hhp($X^a$) ist, wobei $U_3$ und $U_6$ immer ein D-Isomer sind,

(b) Entfernen von zumindest $X^a$ zwecks Entschützens einer primären Seitenketten-Aminogruppe mindestens eines Aminosäurerests des intermediären Peptids;

(c) Umsetzen der entschützten primären Seitenketten-Aminogruppe zwecks Bildens des Rests in einen der Formel U*; und

(d) Abspalten sämtlicher verbleibender Gruppen $X^1$ bis $X^7$ und/oder Abspalten von jeglichem in $X^8$ enthaltenem Harzträger.

**22.** Verfahren nach Anspruch 21, worin entweder $U_5$ U' ist oder $U^6$ U' ist oder beide U' sind.

**Patentansprüche für folgende Vertragsstaaten : ES, GR**

**1.** Verfahren zur Herstellung eines GnRH-Antagonist-Peptid oder eines nichttoxischen Salzes davon, welches Peptid die folgende Formel hat : Ac-$AA_1$-(A)D-Phe-$AA_3$-Ser-AAS-$AA_6$-$AA_7$-AAB-Pro-$AA_{10}$, worin $AA_1$ β-D-NAL, (A)D-Phe oder (B)D-Trp ist; A 4Cl, 4F, $4NO_2$, $4CH_3$, $4OCH_3$, $C^{\alpha}$Me/4Cl, 2,4$Cl_2$, 4Br oder H ist; B H, $6NO_2$, $6NH_2$, $6OCH_3$, 6F, 6Cl, 6Br, $6CH_3$, $N^{in}$For oder $N^{in}$Ac ist; $AA_3$ D-PAL, U*, β-D-NAL oder (B)D-Trp ist; $AA_5$ U*, Lys(cpd) oder Tyr ist; $AA_6$ U*, β-D-NAL, $4NH_2$D-Phe, (B)D-Trp, D-Lys(cpd), D-PAL oder D-Arg ist; $AA_7$ Leu, NML, Nle oder Phe ist; $AA_8$ ILys oder Arg ist; $AA_{10}$ D-Ala-$NH_2$, Gly-$NH_2$, $NHNHCONH_2$ oder NH(R) ist; R Niedrigalkyl ist; und U* entweder

(a)

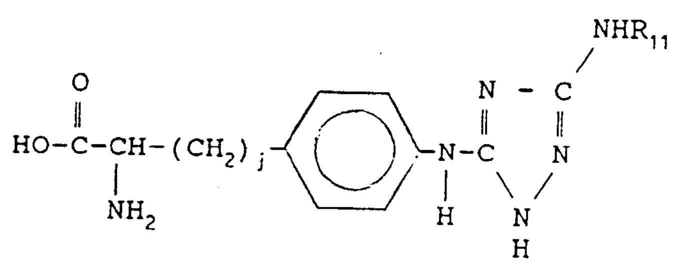

worin j 1 oder 2 und $R_{11}$ H oder ein Acylrest mit 1 bis 6 Kohlenstoffatomen ist;
oder

(b)

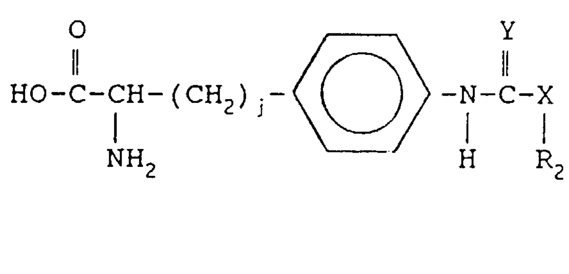

worin j 1, 2 oder 3 ist; X NH oder O ist; Y N-CN oder N-$CONHR_9$ ist, worin $R_9$ H oder Niedrigalkyl ist; $R_2$ Niedrigalkyl, Cyclohexyl, Phenyl, Pyridyl, Methylpyridyl oder Histaminyl ist;

jedoch mit der Maßgabe, daß zumindest eines von $AA_3$, $AA_5$ und $AA_6$ U* ist, wobei AA3 und $AA_6$ immer ein D-Isomer sind,

welches Verfahren umfaßt: (i) Bilden eines intermediären Peptids der Formel: X'-$AA_1$-(A)D-Phe-$U_3$-Ser($X^3$)-$U_5$-$U_6$-$AA_7$-($X^2$ oder $X^7$)-$AA_8$-($X^5$ oder $X^6$)-Pro-$X^8$, worin $U_3$ entweder U' oder $AA_3$ ($X^2$) ist; $U_5$ entweder U' oder $AA_5$($X^4$ oder $X^5$) ist; $U_6$ entweder U' oder $AA_6$($X^4$ oder $X^5$ oder $X^6$) ist; U' Aph($X^a$), Hap($X^a$) oder Hhp($X^a$) ist; $X^1$ Wasserstoff oder eine α-Amino-Schutzgruppe ist; $X^2$ Wasserstoff oder eine Schutzgruppe für einen Indolstickstoff ist; $X^3$ eine Schutzgruppe für eine Hydroxylgruppe von Ser oder Thr ist; $X^4$ Wasserstoff oder eine Schutzgruppe für eine phenolische Hydroxylgruppe von Tyr ist; $X^5$ entweder Wasserstoff oder eine Schutzgruppe für eine Guanidino- oder Imidazolgruppe ist; $X^6$ eine Schutzgruppe für eine primäre Aminoseitenkette ist, von der $X^a$ eine Untergruppe ist, die entfernbar ist, ohne andere Schutzgruppen zu entfernen; $X^7$ Wasserstoff oder eine Schutzgruppe für Met ist; $X^8$ Gly-NH-[Harzträger], D-Ala-NH-[Harzträger], N(A)-[Harzträger], ein Amid entweder von Gly oder von D-Ala oder ein direkt an Pro oder $NHNHCONH_2$ angefügtes substituiertes Amid ist; jedoch mit der Maßgabe, daß zumindest eines von $U_3$, $U_5$ und $U_6$ Aph($X^a$), Hap($X^a$) oder Hhp($X^a$) ist; (ii) Entfernen von zumindest $X^a$ zwecks Entschützens einer primären Seitenketten-Aminogruppe mindestens eines Aminosäurerest des intermediären Peptids; (iii) Umsetzen der entschützten primären Seitenketten-Aminogruppe zwecks Bildens des Rests in einen der Formel U*; (iv) Abspalten sämtlicher verbleibender Gruppen $X^1$ bis $X^7$ und/oder Abspalten von jeglichem in $X^8$ enthaltenem Harzträger, und (v) gegebenenfalls Überführen des Peptids in ein nichttoxisches Salz davon.

2. Verfahren nach Anspruch 1, worin j 1 ist, X NH ist, Y N-CN oder N-$CONH_2$ ist und $R_{11}$ H oder Acetyl ist.

3. Verfahren nach Anspruch 1, worin U* (a) ist, j 1 ist und $R_{11}$ H ist.

4. Verfahren nach Anspruch 3, worin $AA_6$ U* ist.

5. Verfahren nach Anspruch 3, worin $AA_5$ U* ist.

6. Verfahren nach Anspruch 3, worin sowohl $AA_5$ als auch $AA_6$ U* sind.

7. Verfahren nach Anspruch 3, worin $AA_3$ U* ist.

8. Verfahren nach Anspruch 3, worin $AA_1$ β-D-2NAL ist, (A) 4Cl oder 4F ist und $AA_3$ D-3PAL ist.

9. Verfahren nach Anspruch 8, worin $AA_6$ D-Trp, D-PAL, β-D-NAL, (imBzl)D-His oder ($6NO_2$)D-Trp ist und $AA_5$ U* ist.

10. Verfahren nach Anspruch 1, worin das Peptid die folgende Formel hat:
Ac-β-D-2NAL-(4Cl)D-Phe-D-3PAL-Ser-Aph(3-amino-1,2,4-triazol)-D-Aph(3-amino-1,2,4-triazol)-Leu-Lys(isopropyl)-Pro-D-Ala-$NH_2$.

11. Verfahren zur Herstellung eines Peptids oder eines nichttoxischen Salzes davon, welches Peptid die folgende Formel hat:
G-$AA_1$-(A)D-Phe-$AA_3$-Ser-$AA_5$-$AA_6$-$AA_7$-$AA_8$-Pro-$AA_{10}$, worin G Wasserstoff oder eine Acylgruppe mit 7 oder weniger Kohlenstoffatomen ist; $AA_1$ dehydroPro, D-pGlu, (A)D-Phe, (B)D-Trp, Pro oder β-D-NAL ist; A H, Cl, F, $NO_2$, $CH_3$, $OCH_3$, $C^{\alpha}Me/4Cl$, $Cl_2$ oder Br ist; B H, $NO_2$, $NH_2$, $OCH_3$, F, Cl, Br, $CH_3$, $N^{in}$For oder $N^{in}$Ac ist; $AA_3$ U*, D-PAL, β-D-NAL oder (B)D-Trp ist; $AA_5$ U*, Lys(cpd), Orn(cpd), Dbu(cpd), Dpr(cpd), Tyr, (C)Arg, (A) Phe, (3I)Tyr oder His ist; C H oder Niedrigalkyl ist; $AA_6$ U*, β-D-NAL, (B)D-Trp, (A)D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala oder D-Ser(OtBu) ist; A' A, $NH_2$, $NHCH_3$ oder gua ist; D G, cpd oder eine Arylgruppe ist; E H, imBzl oder Dinitrophenol ist; $AA_7$ Nle, Leu, NML, (A) Phe, Met, Nva, Tyr, (B)Trp oder PAL ist; $AA_8$ ILys, (C')Arg oder (C')Har ist; C' H oder Di-Niedrigalkyl ist; $AA_{10}$ D-Ala-$NH_2$, Gly-$NH_2$, $NHNHCONH_2$ oder NH(R) ist; R Niedrigalkyl ist; und U*

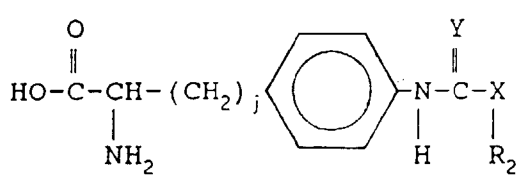

ist, worin j 1, 2 oder 3 ist; Y N-CN, N-$CONHR_9$, O, S oder CH-$NO_2$ ist, worin $R_9$ H, Ac, Alkyl, Naphthyl, Pyridyl,

Pyrimidyl, Pyrazinyl, Indolyl, Chinolinyl oder Imidazolyl ist, welche Alkyl- und zyklischen Gruppen nichtsubstituiert oder substituiert sind;
X NH, O, $N_3$, $M_1(CH_q)_pM_2$ oder $M_1(CH_2)_{p'}$-$M_2(CH_2)_{p''}$-$M_3$ ist, worin $M_1$ $NR_{10}$, N, O oder $CHR_3$ ist, worin $R_3$ Methyl, Ethyl, Propyl, Phenyl, Pyridinyl, Pyrimidinyl oder Purinyl ist; q 1 oder 2 ist; p, p' und p" ganze Zahlen zwischen 0 und 6 sind; $R_{10}$ H, Methyl, Ethyl, Propyl, Phenyl oder substituiertes Phenyl ist; und $M_2$ und $M_3$ $M_1$, COOH, $CONH_2$, $COOR_3$ oder CN sind; $R_1$ H, Alkyl, $(CH_2)_{n'}X''$, Alkenyl, Alkinyl, Aryl oder eine Direktbindung an X ist; n' 1, 2, 3 oder 4 ist; X" $CH_2NH_2$, $CH_2OH$, $CH_2Cl$, $CH_2Br$, $CH_2F$, $CF_3$, oder $CF_2CF_3$ ist; $R_2$ $R_1$, OH, $NH_2$, $NHR_1$, Heterozyklus oder des$R_2$ ist, wobei $R_2$ des$R_2$ ist, wenn X $N_3$ ist; jedoch mit der Maßgabe, daß $R_1$ und $R_2$ gegebenenfalls über eine verzweigte oder unverzweigte Brücke miteinander verbunden sind, so daß $R_1$-$R_2$ entweder -$(CH_2)_m$-oder -$(CH_2)_m$-M-$(CH_2)_{m'}$- ist, wobei m und m' ganze Zahlen von 1 bis 6 sind und M NH, O, S oder $CHR_4$ ist, wobei $R_4$ Niedrigalkyl oder Aryl ist; mit der weiteren Maßgabe, daß Y und $R_2$ gegebenenfalls miteinander verbunden sind; und mit der noch weiteren Maßgabe, daß zumindest eines von $AA_3$, $AA_5$ und $AA_6$ U* ist, wobei U* immer ein D-Isomer ist, wenn es als $AA_3$ oder $AA_8$ anwesend ist, welches Verfahren umfaßt: (i) Bilden eines intermediären Peptids der Formel: $X^1$-$AA_1$-(A)D-Phe-$U_3$-Ser($X^3$)-$U_5$-$U_6$-$AA_7$-($X^2$ oder $X^7$)-$AA_8$-($X^5$ oder $X^6$)-Pro-$X^8$, worin $U_3$ entweder U' oder $AA_3$($X^2$) ist; $U_5$ entweder U' oder $AA_5$($X^4$ oder $X^5$) ist; $U^6$ entweder U' oder $AA_6$($X^4$ oder $X^5$ oder $X^6$) ist; U' Aph($X^a$), Hap($X^a$) oder Hhp($X^a$) ist; $X^1$ Wasserstoff oder eine α-Amino-Schutzgruppe ist; $X^2$ Wasserstoff oder eine Schutzgruppe für einen Indolstickstoff ist; $X^3$ eine Schutzgruppe für eine Hydroxylgruppe von Ser oder Thr ist; $X^4$ Wasserstoff oder eine Schutzgruppe für eine phenolische Hydroxylgruppe von Tyr ist; $X^5$ entweder Wasserstoff oder eine Schutzgruppe für eine Guanidino- oder Imidazolseitenkette ist; $X^6$ eine Schutzgruppe für eine primäre Aminoseitenkette ist, von der $X^a$ eine Untergruppe ist, die entfernbar ist, ohne andere Schutzgruppen zu entfernen; $X^7$ Wasserstoff oder eine Schutzgruppe für Met ist; $X^8$ Gly-NH-[Harzträger], D-Ala-NH-[Harzträger], N(A)-[Harzträger], ein Amid entweder von Gly oder von D-Ala oder ein direkt an Pro oder $NHNHCONH_2$ angefügtes substituiertes Amid ist; jedoch mit der Maßgabe, daß zumindest eines von $U_3$, $U_5$ und $U^6$ Aph($X^a$), Hap($X^a$) oder Hhp ($X^a$) ist; (ii) Entfernen von zumindest $X^a$ zwecks Entschützens einer primären Seitenketten-Aminogruppe mindestens eines Aminosäurerests des intermediären Peptids; (iii) Umsetzen der entschützten primären Seitenketten-Aminogruppe zwecks Bildens des Rests in einen der Formel U*; (iv) Abspalten sämtlicher verbleibender Gruppen $X^1$ bis $X^7$ und/oder Abspalten von jeglichem in $X^8$ enthaltenem Harzträger, und (v) gegebenenfalls Überführen des Peptids in ein nichttoxisches Salz davon.

**12.** Verfahren nach Anspruch 11, worin U* Aph(tcg) ist.

**13.** Verfahren nach Anspruch 11, worin U* Aph(bcg) ist.

**14.** Verfahren nach einem der Ansprüche 11 bis 13, worin $AA_8$ Arg ist, G Ac ist und (D) und (E) beide H sind.

**15.** Verfahren nach Anspruch 11, worin U* einen Cyanoguanidinteil enthält und j 1 ist.

**16.** Verfahren nach Anspruch 11, worin $AA_5$ Aph(tcg) ist, $AA_6$ D-Aph(tcg) ist und $AA_8$ ILys ist.

**17.** Verfahren nach Anspruch 11, worin $AA_5$ Lys(Nic) ist, $AA_6$ U* ist und $AA_8$ ILys ist.

**18.** Verfahren zur Herstellung eines Peptids nach Anspruch 1, worin das Zwischenprodukt die folgende Formel hat: Ac-$AA_1$-(4Cl)D-Phe-$AA_3$-Ser($X^3$)-Aph($X^a$)-D-Aph($X^a$)-Leu-Lys(Ipr,$X^6$)-Pro-D-Ala-NH-[Harzträger], worin $AA_1$ β-D-NAL, (A) D-Phe oder (B)D-Trp ist; B H, $6NO_2$ oder $N^{in}$For ist; $AA_3$ D-PAL, β-D-NAL oder (B)D-Trp ist; $X^3$ eine Schutzgruppe für eine Hydroxylgruppe von Ser oder Thr ist; $X^a$ eine Schutzgruppe für eine primäre Aminogruppe ist, die basenlabil, hydrazinlabil oder thiolabil ist; und $X^6$ Z oder 2ClZ ist.

**19.** Verfahren nach Anspruch 18, worin das Zwischenprodukt die folgende Formel hat:Ac-β-D-2NAL-(4Cl)D-Phe-D-3PAL-Ser($X^3$)-Aph($X^a$)-D-Aph($X^a$)-Leu-Lys(Ipr,A)-Pro-D-Ala-NH-[Harzträger].

**20.** L- oder D-isomere Aminosäure zur Verwendung in einem Verfahren zur Herstellung eines Peptids, welche Aminosäure die folgende Formel hat:

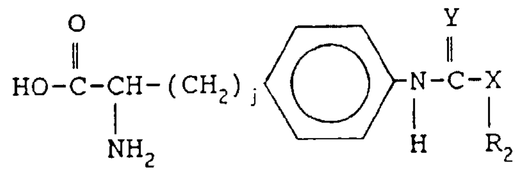

worin j 1,2 oder 3 ist; Y N-CN, N-CONHR$_9$, O, S oder CH-NO$_2$ ist, worin R$_9$ H, Ac, Alkyl, Naphthyl, Pyridyl, Pyrimidyl, Pyrazinyl, Indolyl, Chinolinyl oder Imidazolyl ist, welche Alkyl- und zyklischen Gruppen nichtsubstitiert oder substitutiert sind; X NH, O, N$_3$, $M_1(CH_q)_pM_2$ oder $M_1$-$(CH_2)_{p'}$-$M_2(CH_2)_{p''}$-$M_3$ ist, worin $M_1$ $NR_{10}$, N, O oder $CHR_3$ ist, worin $R_3$ Methyl, Ethyl, Propyl, Phenyl, Pyridinyl, Pyrimidinyl oder Purinyl ist; q 1 oder 2 ist; p, p' und p" ganze Zahlen zwischen 0 und 6 sind; $R_{10}$ H, Methyl, Ethyl, Propyl, Phenyl oder durch Cl, F, $NO_2$, oder $NH_2$ substituiertes Phenyl ist; und $M_2$ und $M_3$ $M_1$, COOH, $CONH_2$, $COOR_3$ oder CN sind; $R_1$ H, Alkyl ($C_1$ bis $C_6$), $(CH_2)_{n'}X''$, Alkenyl ($C_2$ bis $C_4$), Alkinyl ($C_2$ bis $C_4$), Aryl oder eine Direktbindung an X ist; n' 1, 2, 3 oder 4 ist; X" $CH_2NH_2$, $CH_2OH$, $CH_2Cl$, $CH_2Br$, $CH_2F$, $CF_3$ oder $CF_2CF_3$ ist; $R_2$ $R_1$, OH, $NH_2$, $NHR_1$, Heterozyklus oder des$R_2$ ist, wobei $R_2$ des$R_2$ ist, wenn X $N_3$ ist; jedoch mit der Maßgabe, daß $R_1$ und $R_2$ gegebenenfalls über eine verzweigte oder unverzweigte Brücke miteinander verbunden sind, so daß $R_1$-$R_2$entweder -$(CH_2)_m$- oder -$(CH_2)_m$-M-$(CH_2)_{m'}$- ist, wobei m und m' ganze Zahlen von 1 bis 6 sind und M NH, O, S oder $CHR_4$ ist, wobei $R_4$ Niedrigalkyl oder Aryl ist; mit der weiteren Maßgabe, daß Y und $R_2$ gegebenenfalls miteinander verbunden sind.

**Revendications**

**Revendications pour les Etats contractants suivants : AT, BE, CH, DE, DK, FR, GB, IT, LU, NL, SE**

1. Peptide antagoniste de GnRH ou sel non toxique de celui-ci, ledit peptide ayant la formule Ac-AA$_1$-(A)D-Phe-AA$_3$-Ser-AA$_5$-AA$_6$-AA$_7$-AA$_8$-Pro-AA$_{10}$, dans laquelle AA$_1$ est β-D-NAL, (A)D-Phe ou (B)D-Trp ; A est 4Cl, 4F, 4NO$_2$, 4CH$_3$, 4OCH$_3$, C$^{\alpha}$Me/4Cl, 2,4Cl$_2$, 4Br ou H ; B est H, 6NO$_2$, 6NH$_2$, 6OCH$_3$, 6F, 6Cl, 6Br, 6CH$_3$, N$^{in}$For ou N$^{in}$Ac ; AA$_3$ est D-PAL, U*, β-D-NAL ou (B)D-Trp ; AA$_5$ est U*, Lys (cpd) ou Tyr ; AA$_6$ est U*, β-D-NAL, 4NH$_2$D-Phe, (B) D-Trp, D-Lys(cpd), D-PAL ou D-Arg ; AA$_7$ est Leu, NML, Nle ou Phe AA$_8$ est ILys ou Arg ; AA$_{10}$ est D-Ala-NH$_2$, Gly-NH$_2$, NHNHCONH$_2$ ou NH(R) ; R est un groupe alkyle inférieur ; et U* est

(a)

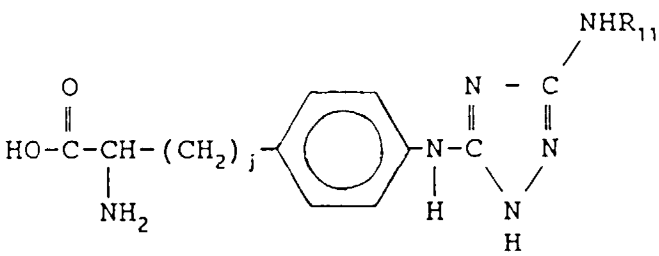

où j vaut 1 ou 2, et R$_{11}$ est H ou un radical acyle ayant de 1 à 6 atomes de carbone ;
ou

(b)

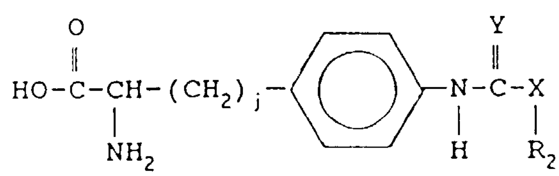

où j vaut 1, 2 ou 3 ; X est NH ou O ; Y est N-CN ou N-CONHR$_9$, R$_9$ étant H ou un groupe alkyle inférieur ; R$_2$ est un groupe alkyle inférieur, cyclohexyle, phényle, pyridyle, méthylpyridyle ou histaminyle ;

à condition toutefois qu'au moins l'un de $AA_3$, $AA_5$ et $AA_6$ soit U*, $AA_3$ et $AA_6$ étant toujours un isomère D.

**2.** Peptide antagoniste de GnRH selon la revendication 1, dans lequel j vaut 1, X est NH, Y est N-CN ou N-$CONH_2$, et $R_{11}$ est H ou un groupe acétyle.

**3.** Peptide selon la revendication 1, dans lequel U* est (a), j vaut 1, et $R_{11}$ est H.

**4.** Peptide selon la revendication 3, dans lequel $AA_6$ est U*.

**5.** Peptide selon la revendication 3, dans lequel $AA_5$ est U*.

**6.** Peptide selon la revendication 3, dans lequel $AA_5$ et $AA_6$ sont tous les deux U*.

**7.** Peptide selon la revendication 3, dans lequel $AA_3$ est U*.

**8.** Peptide selon la revendication 3, dans lequel $AA_1$ est β-D-2NAL, (A) est 4Cl ou 4F, et $AA_3$ est D-3PAL.

**9.** Peptide selon la revendication 8, dans lequel $AA_6$ est D-Trp, D-PAL, β-D-NAL, (imBzl)D-His ou (6$NO_2$)D-Trp, et $AA_5$ est U*.

**10.** Peptide antagoniste de GnRH selon la revendication 1, ayant la formule :
Ac-β-D-2NAL-(4Cl)D-Phe-D-3PAL-Ser-Aph(3-amino-1,2,4-tri-azole)-D-Aph(3-amino-1,2,4-triazole)-Leu-Lys(isopropyl)Pro-D-Ala-$NH_2$.

**11.** Peptide ou sel non toxique de celui-ci, ledit peptide ayant la formule :
G-$AA_1$-(A)D-Phe-$AA_3$-Ser-$AA_5$-$AA_6$-$AA_7$-$AA_8$-Pro-$AA_{10}$,
dans laquelle G est l'hydrogène ou un groupe acyle ayant 7 atomes de carbone ou moins ; $AA_1$ est déhydroPro, D-pGlu,

(A)D-Phe, (B)D-Trp, Pro ou β-D-NAL ; A est H, Cl, F, $NO_2$, $CH_3$, $OCH_3$, $C^{\alpha}$Me/4Cl, $Cl_2$ ou Br ; B est H, $NO_2$, $NH_2$, $OCH_3$, F, Cl, Br, $CH_3$, $N^{in}$For ou $N^{in}$Ac ; $AA_3$ est U*, D-PAL, β-D-NAL ou (B)D-Trp ; $AA_5$ est U*, Lys(cpd), Orn(cpd), Dbu(cpd), Dpr(cpd), Tyr, (C)Arg, (A)Phe, (3I)Tyr ou His ; C est H ou un groupe alkyle inférieur ; $AA_6$ est U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala ou D-Ser(OtBu) ; A' est A, $NH_2$, $NHCH_3$ ou gua ; D est G, cpd ou un groupe aryle ; E est H, imBzl ou dinitrophénol ; $AA_7$ est Nle, Leu, NML, (A)Phe, Met, Nva, Tyr, (B)Trp, ou PAL ; $AA_8$ est ILys (C')Arg ou (C')Har ; C' est H ou un groupe dialkyle inférieur ; $AA_{10}$ est D-Ala-$NH_2$, Gly-$NH_2$, $NHNHCONH_2$ ou NH(R) ; R est un groupe alkyle inférieur ; et U* est

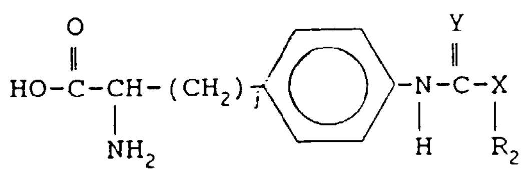

où j = 1, 2 ou 3 ; Y est N-CN, N-$CONHR_9$, O, S ou CH-$NO_2$, $R_9$ étant H, Ac, un groupe alkyle, naphtyle, pyridyle, pyrimidyle, pyrazinyle, indolyle, quinolinyle ou imidazolyle, les groupes alkyle et les groupes cycliques étant substitués ou non substitués ; X est NH, O, $N_3$, $M_1(CH_q)_pM_2$ ou $M_1$-$(CH_2)_{p'}$-$M_2(CH_2)_{p''}$-$M_3$, où $M_1$ est $NR_{10}$, N, O ou $CHR_3$ où $R_3$ est un groupe méthyle, éthyle, propyle, phényle, pyridinyle, pyrimidinyle ou purinyle ; q vaut 1 ou 2 ; p, p' et p" sont des nombres entiers valant entre 0 et 6 ; $R_{10}$ est H ou un groupe méthyle, éthyle, propyle, phényle ou phényle substitué ; et $M_2$ et $M_3$ sont $M_1$, COOH, $CONH_2$, $COOR_3$ ou CN ; $R_1$ est H, un groupe alkyle, $(CH_2)_{n'}X''$, alcényle, alkynyle, aryle ou une liaison directe avec X ; n' vaut 1, 2, 3 ou 4 ; X" est $CH_2NH_2$, $CH_2OH$, $CH_2Cl$, $CH_2Br$, $CH_2F$, $CF_3$ ou $CF_2CF_3$ ; $R_2$ est $R_1$, OH, $NH_2$, $NHR_1$, un hétérocycle ou des$R_2$, $R_2$ étant des$R_2$ quand X est $N_3$ ; à condition toutefois que $R_1$ et $R_2$ soient éventuellement reliés au moyen d'un pont ramifié ou non ramifié, de sorte que $R_1$-$R_2$ est -$(CH_2)_m$- ou -$(CH_2)_m$-M-$(CH_2)_{m'}$- où m et m' sont des nombres entiers de 1 à 6, et M est NH, O, S ou $CHR_4$, $R_4$ étant un groupe alkyle inférieur ou aryle ; à condition aussi que Y et $R_2$ soient éventuellement reliés ; et à condition aussi qu'au moins l'un de $AA_3$, $AA_5$ et $AA_6$ soit

U*, U* étant un isomère D quand il est présent en tant que $AA_3$ ou $AA_6$.

**12.** Peptide selon la revendication 11, dans lequel U* est Aph(tcg).

**13.** Peptide selon la revendication 11, dans lequel U* est Aph(bcg).

**14.** Peptide selon l'une quelconque des revendications 11 à 13, dans lequel $AA_8$ est Arg, G est Ac, et (D) et (E) sont tous les deux H.

**15.** Peptide selon la revendication 11, dans lequel U* contient un fragment cyanoguanidine et dans lequel j vaut 1.

**16.** Peptide selon la revendication 11, dans lequel $AA_5$ est Aph(tcg), $AA_6$ est D-Aph(tcg) et $AA_8$ est ILys.

**17.** Peptide selon la revendication 11, dans lequel $AA_5$ est Lys(Nic), $AA_6$ est U* et $AA_8$ est ILys.

**18.** Intermédiaire peptidique pour la préparation d'un peptide selon la revendication 1, ledit intermédiaire ayant la formule : Ac-$AA_1$-(4Cl)D-Phe-$AA_3$-Ser($X^3$)-Aph($X^a$)-N-Aph ($X^a$)-Leu-Lys (Ipr,$X^6$)-Pro-D-Ala-NH-[support de résine], dans laquelle $AA_1$ est β-D-NAL, (A)D-Phe ou (B)D-Trp ; B est H, $6NO_2$ ou $N^{in}$For ; $AA_3$ est D-PAL, β-D-PAL ou (B)D-Trp ; $X^3$ est un groupe protecteur pour un groupe hydroxyle de Ser ou de Thr ; $X^a$ est un groupe protecteur pour un groupe amino primaire qui est instable vis-à-vis d'une base, instable vis-à-vis de l'hydrazine ou instable vis-à-vis d'un groupe thio ; et $X^6$ est Z ou 2ClZ.

**19.** Intermédiaire peptidique selon la revendication 18, ayant la formule : Ac-β-D-2NAL-(4Cl)D-Phe-D-3PAL-Ser($X^3$)-Aph($X^a$)-D-Aph($X^a$)-Leu-Lys (Ipr,Z)-Pro-D-Ala-NH-[support de résine].

**20.** Amino-acide isomère L ou D non naturel ayant la formule :

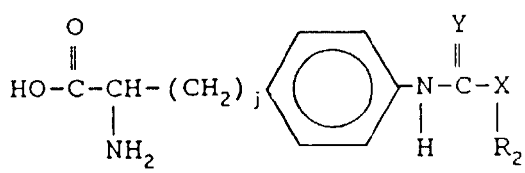

dans laquelle j vaut 1, 2 ou 3 ; Y est N-cN, N-$CONHR_9$, O, S ou CH-$NO_2$, où $R_9$ est H, Ac, un groupe alkyle, naphtyle, pyridyle, pyrimidyle, pyrazinyle, indolyle, quinolinyle ou imidazolyle, lesdits groupes alkyle et cycliques étant substitués ou non substitués ; X est NH, O, $N_3$, $M_1(CH_q)_pM_2$ ou $M_1$-$(CH_2)_{p'}$-$M_2(CH_2)_{p''}$-$M_3$, où $M_1$ est $NR_{10}$, N, O ou $CHR_3$ où $R_3$ est un groupe méthyle, éthyle, propyle, phényle, pyridinyle, pyrimidinyle ou purinyle ; q vaut 1 ou 2 ; p, p' et p" sont des nombres entiers valant entre 0 et 6 ; $R_{10}$ est H ou un groupe méthyle, éthyle, propyle, phényle ou phényle substitué par Cl, F, $NO_2$, ou $NH_2$ ; et $M_2$ et $M_3$ sont $M_1$, COOH, $CONH_2$, $COOR_3$ ou CN ; $R_1$ est H, un groupe alkyle (en $C_1$ à $C_6$), $(CH_2)_nX$", alcényle (en $C_2$ à $C_4$), t alkynyle (en $C_2$ à $C_4$), aryle ou une liaison directe avec X ; n' vaut 1, 2, 3 ou 4 ; X" est $CH_2NH_2$, $CH_2OH$, $CH_2Cl$, $CH_2Br$, $CH_2F$, $CF_3$ ou $CF_2CF_3$ ; $R_2$ est $R_1$, OH, $NH_2$, $NHR_1$, un hétérocycle ou des$R_2$, $R_2$ étant des$R_2$ quand X est $N_3$ ; à condition toutefois que $R_1$ et $R_2$ soient éventuellement reliés au moyen d'un pont ramifié ou non ramifié, de sorte que $R_1$-$R_2$ est $-(CH_2)_m-$ ou $-(CH_2)_m$-M-$(CH_2)_{m'}$- où m et m' sont des nombres entiers de 1 à 6, et M est NH, O, S ou $CHR_4$, $R_4$ étant un groupe alkyle inférieur ou aryle ; à condition aussi que Y et $R_2$ soient éventuellement reliés.

**21.** Procédé de préparation d'un peptide comprenant un résidu U* d'un amino-acide non naturel selon la revendication 20, ledit peptide ayant la formule : G-$AA_1$-(A)D-Phe-$AA_3$-Ser-$AA_5$-$AA_6$-$AA_7$-$AA_8$-Pro-$AA_{10}$, dans laquelle G est l'hydrogène ou un groupe acyle ayant 7 atomes de carbone ou moins ; $AA_1$ est déhydroPro, D-pGlu, (A)D-Phe, (B)D-Trp, Pro ou β-D-NAL ; A est H, Cl, F, $NO_2$, $CH_3$, $OCH_3$, $C^{\alpha}$ Me/4Cl, $Cl_2$ ou Br ; B est H, $NO_2$, $NH_2$, $OCH_3$, F, Cl, Br, $CH_3$, $N^{in}$For ou $N^{in}$Ac ; $AA_3$ est U*, D-PAL, β-D-NAL ou (B)D-Trp ; $AA_5$ est U*, Lys(cpd), Orn(cpd), Dbu(cpd), Dpr(cpd), Tyr, (C)Arg, (A)Phe, (3I)Tyr ou His ; C est H ou un groupe alkyle inférieur ; $AA_6$ est U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (c)D-Arq, D-Leu, D-Ile, D-Nle, D-Val, D-Ala ou D-Ser(OtBu) ; A' est A, $NH_2$, $NHCH_3$ ou gua ; D est G, cpd ou un groupe aryle ; E est H, imBzl ou dinitrophénol ; $AA_7$ est Nle, Leu, NML, (A)Phe, Met, Nva, Tyr, (B)Trp, ou PAL ; $AA_8$ est ILys (C')Arg ou (C')Har ; C' est H ou un groupe dialkyle inférieur ; $AA_{10}$ est D-Ala-$NH_2$, Gly-$NH_2$, $NHNHCONH_2$ ou NH(R) ; R est un groupe

alkyle inférieur ; à condition toutefois qu'au moins l'un de $AA_3$, $AA_5$ et $AA_6$ soit U*, ledit procédé comprenant (a) la formation d'un peptide intermédiaire ayant la formule : $X^1$-$AA_1$-(A)D-Phe-$U_3$-Ser($X^3$)-$U_5$-$U_6$-$AA_7$($X^2$ ou $X^7$)-$AA_8$ ($X^5$ ou $X^6$)-Pro-$X^8$ dans laquelle $U_3$ est U' ou $AA_3$($X^2$) ; $U_5$ est U' ou $AA_5$($X^4$ ou $X^5$) ; $U^6$ est U' ou $AA_6$($X^4$ ou $X^5$ ou $X^6$) ; U' est Aph($X^a$), Hap($X^a$) ou Hhp($X^a$) ; $X^1$ est l'hydrogène ou un groupe protecteur pour un groupe $\alpha$-amino ; $X^2$ est l'hydrogène ou un groupe protecteur pour un atome d'azote d'indole ; $X^3$ est un groupe protecteur pour un groupe hydroxyle de Ser ou de Thr ; $X^4$ est l'hydrogène ou un groupe protecteur pour un groupe hydroxyle phénolique de Tyr ; $X^5$ est l'hydrogène ou un groupe protecteur pour un groupe guanidino ou imidazole ; $X^6$ est un groupe protecteur pour un groupe amino primaire ; $X^a$ est un groupe protecteur pour un groupe amino primaire qui est instable vis-à-vis d'une base, instable vis-à-vis de l'hydrazine ou instable vis-à-vis d'un groupe thio ; $X^7$ est l'hydrogène ou un groupe protecteur pour Met ; $X^8$ est Gly-NH-[support de résine] , D-Ala-NH-[support de résine], N(A)-[support de résine], un amide de Gly ou de D-Ala ou un amide substitué attaché directement à Pro ; à condition toutefois qu'au moins l'un de $U_3$, $U_5$ et $U_6$ soit Aph($X^a$), Hap($X^a$) ou Hhp($X^a$), $U_3$ et $U_6$ étant toujours un isomère D ; (b) l'élimination d'au moins un $X^a$ pour déprotéger un groupe amino primaire de la chaîne latérale d'au moins un résidu amino-acide dudit peptide intermédiaire ; (c) la mise en réaction dudit groupe amino primaire déprotégé de la chaîne latérale pour transformer ledit résidu en un résidu ayant la formule U* ; et (d) la séparation par clivage de tous les groupes $X^1$ à $X^7$ restants et/ou le clivage de tout support de résine inclus dans $X^8$.

**22.** Procédé selon la revendication 21, dans lequel $U_5$ est U', ou $U_6$ est U', ou $U_5$ et $U_6$ sont tous les deux U'.

**Revendications pour les Etats contractants suivants : ES, GR**

**1.** Procédé de préparation d'un peptide antagoniste de GnRH ou d'un sel non toxique de celui-ci, ledit peptide ayant la formule : Ac-$AA_1$-(A)D-Phe-$AA_3$-Ser-$AA_5$-$AA_6$-$AA_7$-$AA_8$-Pro-$AA_{10}$, dans laquelle $AA_1$ est $\beta$-D-NAL, (A)D-Phe ou (B)D-Trp ; A est 4Cl, 4F, $4NO_2$, $4CH_3$, $4OCH_3$, $C^{\alpha}Me/4Cl$, $2,4Cl_2$, 4Br ou H ; B est H, $6NO_2$, $6NH_2$, $6OCH_3$, 6F, 6Cl, 6Br, $6CH_3$, $N^{in}For$ ou $N^{in}Ac$ ; $AA_3$ est D-PAL, U*, $\beta$-D-NAL ou (B)D-Trp ; $AA_5$ est U*, Lys(cpd) ou Tyr ; $AA_6$ est U*, $\beta$-D-NAL, $4NH_2$D-Phe, (B)D-Trp, D-Lys(cpd), D-PAL ou D-Arg ; $AA_7$ est Leu, NML, Nle ou Phe ; $AA_8$ est ILys ou Arg ; $AA_{10}$ est D-Ala-$NH_2$, Gly-$NH_2$, $NHNHCONH_2$ ou NH(R) ; R est un groupe alkyle inférieur ; et U* est

(a)

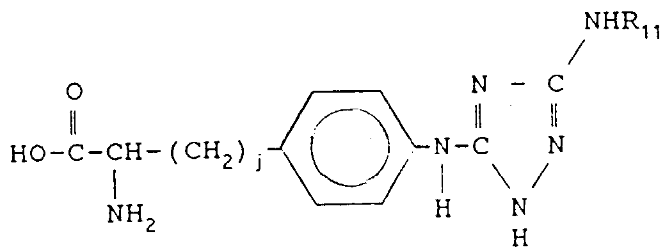

où j vaut 1 ou 2, et $R_{11}$ est H ou un radical acyle ayant de 1 à 6 atomes de carbone ;
ou

(b)

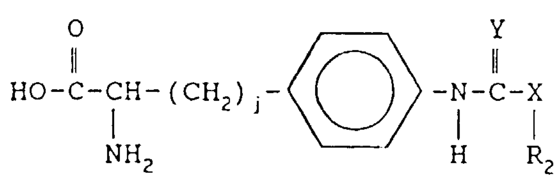

où j vaut 1, 2 ou 3 ; X est NH ou O ; Y est N-CN ou N-$CONHR_9$, $R_9$ étant H ou un groupe alkyle inférieur ; $R_2$ est un groupe alkyle inférieur, cyclohexyle, phényle, pyridyle, méthylpyridyle ou histaminyle ;

à condition toutefois qu'au moins l'un de $AA_3$, $AA_5$ et $AA_6$ soit U*, $AA_3$ et $AA_6$, étant toujours un isomère D, ledit procédé comprenant (i) la formation d'un peptide intermédiaire ayant la formule : $X^1$-$AA_1$-(A)P-Phe-$U_3$-Ser ($X^3$)-$U_5$-$U_6$-$AA_7$($X^2$ ou $X^7$)-$AA_8$($X^5$ ou $X^6$)-Pro-$X^8$ dans laquelle $U_3$ est U' ou $AA_3$($X^2$) ; $U_5$ est U' ou $AA_5$($X^4$ ou $X^5$); $U^6$ est U' ou $AA_6$($X^4$ ou $X^5$ ou $X^6$) ; U' est Aph($X^a$), Hap($X^a$) ou Hhp($X^a$); $X^1$ est l'hydrogène ou un groupe protecteur

pour un groupe α-amino ; $X^2$ est l'hydrogène ou un groupe protecteur pour un atome d'azote d'indole ; $X^3$ est un groupe protecteur pour un groupe hydroxyle de Ser ou de Thr ; $X^4$ est l'hydrogène ou un groupe protecteur pour un groupe hydroxyle phénolique de Tyr ; $X^5$ est l'hydrogène ou un groupe protecteur pour une chaîne latérale guanidino ou imidazole ; $X^6$ est un groupe protecteur pour une chaîne latérale amino primaire dont $X^a$ est un sous-groupe qu'on peut enlever sans enlever d'autres groupes protecteurs ; $X^7$ est l'hydrogène ou un groupe protecteur pour Met ; $X^8$ est Gly-NH-[support de résine], D-Ala-NH-[support de résine], N(A)-[support de résine], un amide de Gly ou de D-Ala ou un amide substitué attaché directement à Pro ou à $NHNHCONH_2$ ; à condition toutefois qu'au moins l'un de $U_3$, $U_5$ et $U_6$ soit Aph($X^a$), Hap($X^a$) ou Hhp($X^a$) ; (ii) l'élimination d'au moins un $X^a$ pour déprotéger un groupe amino primaire de la chaîne latérale d'au moins un résidu amino-acide dudit peptide intermédiaire ; (iii) la mise en réaction dudit groupe amino primaire déprotégé de la chaîne latérale pour transformer ledit résidu en un résidu ayant la formule U* ; (iv) la séparation par clivage de tous les groupes $X^1$ à $X^7$ restants et/ou le clivage de tout support de résine inclus dans $X^8$, et (v) le cas échéant, la transformation dudit peptide en un sel non toxique de celui-ci.

**2.** Procédé selon la revendication 1, dans lequel j vaut 1, X est NH, Y est N-CN ou N-$CONH_2$, et $R_{11}$ est H ou un groupe acétyle.

**3.** Procédé selon la revendication 1, dans lequel U* est (a), j vaut 1, et $R_{11}$ est H.

**4.** Procédé selon la revendication 3, dans lequel $AA_6$ est U*.

**5.** Procédé selon la revendication 3, dans lequel $AA_5$ est U*.

**6.** Procédé selon la revendication 3, dans lequel $AA_5$ et $AA_6$ sont tous les deux U*.

**7.** Procédé selon la revendication 3, dans lequel $AA_3$ est U*.

**8.** Procédé selon la revendication 3, dans lequel $AA_1$ est β-D-2NAL, (A) est 4Cl ou 4F, et $AA_3$ est D-3PAL.

**9.** Procédé selon la revendication 8, dans lequel $AA_6$ est D-Trp, D-PAL, β-D-NAL, (imBzl)D-His ou ($6NO_2$)D-Trp, et $AA_5$ est U*.

**10.** Procédé selon la revendication 1, dans lequel ledit peptide a la formule : Ac-β-D-2NAL-(4Cl)D-Phe-D-3PAL-Ser-Aph(3-amino-1,2,4-triazole)-D-Aph(3-amino-1,2,4-triazole)-Leu-Lys (isopropyl)-Pro-D-Ala-$NH_2$.

**11.** Procédé de préparation d'un peptide ou d'un sel non toxique de celui-ci, ledit peptide ayant la formule : G-$AA_1$-(A)D-Phe-$AA_3$-Ser-$AA_5$-$AA_6$-$AA_7$-$AA_8$-Pro-$AA_{10}$, dans laquelle G est l'hydrogène ou un groupe acyle ayant 7 atomes de carbone ou moins ; $AA_1$ est déhydroPro, D-pGlu, (A)D-Phe, (B)D-Trp, Pro ou β-D-NAL ; A est H, Cl, F, $NO_2$, $CH_3$, $OCH_3$, $C^{\alpha}Me$/4Cl, $Cl_2$ ou Br ; B est H, $NO_2$, $NH_2$, $OCH_3$, F, Cl, Br, $CH_3$, $N^{in}$For ou $N^{in}$Ac ; $AA_3$ est U*, D-PAL, β-D-NAL ou (B)D-Trp ; $AA_5$ est U*, Lys(cpd), Orn(cpd), Dbu(cpd), Dpr(cpd), Tyr, (C)Arg, (A)Phe, (3I)Tyr ou His ; C est H ou un groupe alkyle inférieur ; $AA_6$ est U*, β-D-NAL, (B)D-Trp, (A')D-Phe, (D)D-Orn, (D)D-Lys, (D)Dbu, (D)D-Dpr, D-Har, D-Tyr, (E)D-His, D-PAL, (C)D-Arg, D-Leu, D-Ile, D-Nle, D-Val, D-Ala ou D-Ser(OtBu) ; A' est A, $NH_2$, $NHCH_3$ ou gua ; D est G, cpd ou un groupe aryle ; E est H, imBzl ou dinitrophénol; $AA_7$ est Nle, Leu, NML, (A)Phe, Met, Nva, Tyr, (B)Trp ou PAL ; $AA_8$ est ILys (C')Arg ou (C')Har ; C' est H ou un groupe dialkyle inférieur ; $AA_{10}$ est D-Ala-$NH_2$, Gly-$NH_2$, $NHNHCONH_2$ ou NH(R) ; R est un groupe alkyle inférieur ; et U* est

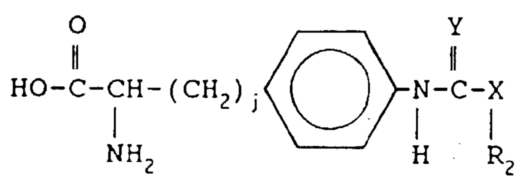

où j = 1, 2 ou 3 ; Y est N-CN, N-$CONHR_9$, O, S ou CH-$NO_2$, $R_9$ étant H, Ac, un groupe alkyle, naphtyle, pyridyle, pyrimidyle, pyrazinyle, indolyle, quinolinyle ou imidazolyle, les groupes alkyle et les groupes cycliques étant substitués ou non substitués ; X est NH, O, $N_3$, $M_1(CH_q)_pM_2$ ou $M_1$-$(CH_2)_{p'}$-$M_2(CH_2)_{p''}$-$M_3$, où $M_1$ est $NR_{10}$, N, O ou $CHR_3$ où $R_3$ est un groupe méthyle, éthyle, propyle, phényle, pyridinyle, pyrimidinyle ou purinyle ; q vaut 1 ou 2 ;

p, p' et p" sont des nombres entiers valant entre 0 et 6 ; $R_{10}$ est H ou un groupe méthyle, éthyle, propyle, phényle ou phényle substitué ; et $M_2$ et $M_3$ sont $M_1$, COOH, $CONH_2$, $COOR_3$ ou CN ; $R_1$ est H, un groupe alkyle, $(CH_2)_{n'}X"$, alcényle, alkynyle, aryle ou une liaison directe avec X ; n' vaut 1, 2, 3 ou 4 ; X" est $CH_2NH_2$, $CH_2OH$, $CH_2Cl$, $CH_2Br$, $CH_2F$, $CF_3$ ou $CF_2CF_3$ ; $R_2$ est $R_1$, OH, $NH_2$, $NHR_1$, un hétérocycle ou des$R_2$, $R_2$ étant des$R_2$ quand X est $N_3$ ; à condition toutefois que $R_1$ et $R_2$ soient éventuellement reliés au moyen d'un pont ramifié ou non ramifié, de sorte que $R_1$-$R_2$ est $-(CH_2)_m-$ ou $-(CH_2)_m-M-(CH_2)_{m'}-$ où m et m' sont des nombres entiers de 1 à 6, et M est NH, O, S ou $CHR_4$, $R_4$ étant un groupe alkyle inférieur ou aryle ; à condition aussi que Y et $R_2$ soient éventuellement reliés ; et à condition aussi qu'au moins l'un de $AA_3$, $AA_8$ et $AA_6$ soit U*, U* étant un isomère D quand il est présent en tant que $AA_3$ ou $AA_6$ ;

ledit procédé comprenant (1) la formation d'un peptide intermédiaire ayant la formule : $X^1$-$AA_1$-(A)N-Phe-$N_3$-Ser($X^3$)-$U_5$-$U_6$-$AA_7$($X^2$ ou $X^7$)-$AA_8$($X^5$ ou $X^6$)-Pro-$X^8$ dans laquelle $U_3$ est U' ou $AA_3$($X^2$) ; $U_3$ est U' ou $AA_5$($X^4$ ou $X^5$) ; $U_6$ est U' ou $AA_6$($X^4$ ou $X^5$ ou $X^6$) ; U' est Aph($X^a$), Hap($X^a$) ou Hhp($X^a$) ; $X^1$ est l'hydrogène ou un groupe protecteur pour un groupe $\alpha$-amino ; $X^2$ est l'hydrogène ou un groupe protecteur pour un atome d'azote d'indole ; $X^3$ est un groupe protecteur pour un groupe hydroxyle de Ser ou de Thr ; $X^4$ est l'hydrogène ou un groupe protecteur pour un groupe hydroxyle phénolique de Tyr ; $X^5$ est l'hydrogène ou un groupe protecteur pour une chaîne latérale guanidino ou imidazole ; $X^6$ est un groupe protecteur pour une chaîne latérale amino primaire dont $X^a$ est un sous-groupe qu'on peut enlever sans enlever d'autres groupes protecteurs ; $X^7$ est l'hydrogène ou un groupe protecteur pour Met ; $X^8$ est Gly-NH-[support de résine] , D-Ala-NH-[support de résine], N(A)-[support de résine], un amide de Gly ou de D-Ala ou un amide substitué attaché directement à Pro ou à $NHNHCONH_2$ ; à condition toutefois qu'au moins l'un de $U_3$, $U_5$ et $U_6$ soit Aph($X^a$), Hap($X^a$) ou Hhp($X^a$) ; (ii) l'élimination d'au moins un $X^a$ pour déprotéger un groupe amino primaire de la chaîne latérale d'au moins un résidu amino-acide dudit peptide intermédiaire ; (iii) la mise en réaction dudit groupe amino primaire déprotégé de la chaîne latérale pour transformer ledit résidu en un résidu ayant la formule U* ; (iv) la séparation par clivage de tous les groupes $X^1$ à $X^7$ restants et/ou le clivage de tout support de résine inclus dans $X^8$, et (v) le cas échéant, la transformation dudit peptide en un sel non toxique de celui-ci.

**12.** Procédé selon la revendication 11, dans lequel U* est Aph(tcg).

**13.** Procédé selon la revendication 11, dans lequel U* est Aph(bcg).

**14.** Procédé selon l'une quelconque des revendications 11 à 13, dans lequel $AA_8$ est Arg, G est Ac, et (D) et (E) sont tous les deux H.

**15.** Procédé selon la revendication 11, dans lequel U* contient un fragment cyanoguanidine et dans lequel j vaut 1.

**16.** Procédé selon la revendication 11, dans lequel $AA_5$ est Aph(tcg), $AA_6$ est D-Aph(tcg) et $AA_8$ est ILys.

**17.** Procédé selon la revendication 11, dans lequel $AA_5$ est Lys(Nic), $AA_6$ est U* et $AA_8$ est ILys.

**18.** Procédé de préparation d'un peptide selon la revendication 1, ledit intermédiaire ayant la formule : Ac-$AA_1$-(4Cl)D-Phe-$AA_3$-Ser($X_3$)-Aph($X^a$)-D-Aph($X^a$)-Leu-Lys(Ipr,$X^6$)-Pro-D-Ala-NH-[support de résine], dans laquelle $AA_1$ est $\beta$-D-NAL, (A)D-Phe ou (B)D-Trp ; B est H, $6NO_2$ ou $N^{in}$For ; $AA_3$ est D-PAL, $\beta$-D-PAL ou (B)D-Trp ; $X^3$ est un groupe protecteur pour un groupe hydroxyle de Ser ou de Thr ; $X^a$ est un groupe protecteur pour un groupe amino primaire qui est instable vis-à-vis d'une base, instable vis-à-vis de l'hydrazine ou instable vis-à-vis d'un groupe thio ; et $X^6$ est Z ou 2ClZ.

**19.** Procédé selon la revendication 18, dans lequel ledit intermédiaire a la formule : Ac-$\beta$-D-2NAL-(4Cl)D-Phe-D-3PAL-Ser($X^3$)-Aph($X^a$)-D-Aph($X^a$)-Leu-Lys(Ipr,Z)-Pro-D-Ala-NH-[support de résine].

**20.** Amino-acide isomère L ou D destiné à être utilisé dans un procédé de préparation d'un peptide, ledit amino-acide ayant la formule :

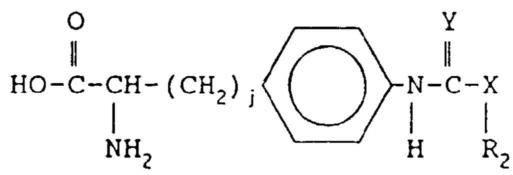

dans laquelle j vaut 1, 2 ou 3 ; Y est N-CN, N-CONHR$_9$, O, S ou CH-NO$_2$, où R$_9$ est H, Ac, un groupe alkyle, naphtyle, pyridyle, pyrimidyle, pyrazinyle, indolyle, quinolinyle ou imidazolyle, lesdits groupes alkyle et cycliques étant substitués ou non substitués ; X est NH, O, N$_3$, $M_1(CH_q)_pM_2$ ou $M_1$-$(CH_2)_{p'}$-$M_3(CH_2)_{p''}$-$M_3$, où $M_1$ est $NR_{10}$, N, O ou $CHR_3$ où $R_3$ est un groupe méthyle, éthyle, propyle, phényle, pyridinyle, pyrimidinyle ou purinyle ; q vaut 1 ou 2 ; p, p' et p" sont des nombres entiers valant entre O et 6 ; $R_{10}$ est H ou un groupe méthyle, éthyle, propyle, phényle ou phényle substitué par Cl, F, $NO_2$, ou $NH_2$ ; et $M_2$ et $M_3$ sont $M_1$, COOH, $CONH_2$, $COOR_3$ ou CN ; $R_1$ est H, un groupe alkyle (en $C_1$ à $C_6$), $(CH_2)_{n'}X"$, alcényle (en $C_2$ à $C_4$), alkynyle (en $C_2$ à $C_4$), aryle ou une liaison directe avec X ; n' vaut 1, 2, 3 ou 4 ; X" est $CH_2NH_2$, $CH_2OH$, $CH_2Cl$, $CH_2Br$, $CH_2F$, $CF_3$ ou $CF_2CF_3$ ; $R_2$ est $R_1$, OH, $NH_2$, $NHR_1$, un hétérocycle ou des$R_2$, $R_2$ étant des$R_2$ quand X est $N_3$ ; à condition toutefois que $R_1$ et $R_2$ soient éventuellement reliés au moyen d'un pont ramifié ou non ramifié, de sorte que $R_1$-$R_2$ est $-(CH_2)_m-$ ou $-(CH_2)_m-M-(CH_2)_{m'}$ où m et m' sont des nombres entiers de 1 à 6, et M est NH, O, S ou $CHR_4$, $R_4$ étant un groupe alkyle inférieur ou aryle ; à condition aussi que Y et $R_2$ soient éventuellement reliés.